# EUROPEAN PATENT APPLICATION

(11) **EP 4 134 078 A1**
(43) Date of publication of application: **15.02.2023**
(21) Application number: 21784376.2
(22) Date of filing: 09.04.2021
(51) Int. Cl.: A61K 31/4045, A61K 9/08, A61K 47/10, A61P 31/14

(54) **INJECTABLE MELATONIN COMPOSITION FOR THE TREATMENT OF VIRAL DISEASES**

(30) Priority: 09.04.2020 WO PCT/ES2020/070234
(71) Applicant: Universidad de Granada, 18071 Granada (ES)
(72) Inventor: ESCAMES ROSA, Germaine, 18071 Granada (ES); ACUÑA CASTROVIEJO, Darío, 18071 Granada (ES)
(74) Representative: Hoffmann Eitle
(86) International application number: PCT/ES2021/070235
(87) International publication number: WO 2021/205053

(57) **Abstract**

The present invention comprises an aqueous melatonin composition with unexpected long-term stability that allows high concentrations of this water-insoluble active substance for use thereof as an antiviral treatment.

## Description

### FIELD OF THE INVENTION

The present invention is comprised in the field of medicine and pharmacy, and relates to an injectable melatonin composition having a high stability for the treatment of viral diseases. In particular, the present invention relates to the use of said composition in the treatment of patients infected with SARS-CoV-2.

### STATE OF THE ART

In the face of a pandemic like the current COVID-19 pandemic, the questions of how to combat these threats and how to reduce fatality in infected persons are crucial. Solutions are particularly urgent when specific therapies are not available. Vaccination requires time for development and is not immediately applicable. Even for coronaviruses that have been known for a long time, their genetic variability leads to several subtypes with different appearances from year to year, such as seasonal flu, requiring repeated vaccine adaptations.

The alternative to vaccine development requires the identification of a drug which controls the virus. To that end, it is necessary for the potential antiviral medication to interfere with intracellular processes such as binding by host cell proteases, interference with the fusion of the viral envelope with host cell membranes, virus replication, or virion release. In the case of COVID-19, and up until now, there are major limitations in identifying antiviral medications which control the virus.

The anti-malarial drug, chloroquine, is under study with findings awaiting verification, with the aggravating circumstance of its undesirable side effects (Gao J, Tian Z, Yang X (2020) Breakthrough: Chloroquine phosphate has shown apparent efficacy in treatment of COVID-19 associated pneumonia in clinical studies. BioSci. Trends 4 (1): 72-73. DOI: 10. 5582/bst.2020.01047.). Camostat mesylate, an inhibitor of the host cell surface protease required for the endocytic entry of the SARSCoV-2 virus after its binding, also requires further research (Hoffmann M, et al. (2020) SARS-CoV-2 cell entry depends on ACE2 and TMPRSS2 and is blocked by a clinically-proven protease inhibitor. Cell. DOI: 10.1016/j.cell.2020.02,052).

Melatonin, among others, has been identified as a potential anti-HCoV drug in a recent publication on antiviral drug repurposing, and by means of quantifying the interaction between the HCoV-host interactome and drug targets in the human protein-protein interaction network (Network-based drug repurposing for novel coronavirus 2019-nCoV/SARS-CoV-2) (Zhou Y., Hou Y, Shen J, Huan Y, Martin W, Feixiong Cheng F (2020). Network-based drug repurposing for novel coronavirus 2019-nCoV/SARS-Co. Cell Discovery. DOl.org/10.1038/s41421-020-0153-3).

The analysis of melatonin as a potential drug for the COVID-19 pandemic must be conducted from the perspective of controlling host responses in the event of viral infection, particularly with respect to the severe progressions of the disease, given that these inflammatory responses are often fatal. It should be noted that the high mortality of infectious viral diseases such as COVID-19, SARS, Ebola, MERS, or avian flu is caused by an uncontrolled innate immune response with destructive inflammation (Tan DX, Hardeland R (2020). Potential utility of melatonin in deadly infectious diseases related to the overreaction of innate immune response and destructive inflammation: focus on COVID-19. Melatonin Research. 3, 120-143. DOl.org/10.32794/mr11250052).

The mechanisms underlying the high mortality of said viral infections involve attack by free radicals and destructive inflammation in various tissues and organs. Just as the main effector in Ebola is the vascular system, in the case of COVID-19, the main effector is the lung. Although responses are linked to a damaged host innate immune system, in some cases, a direct viral contribution to inflammation was evident. For example, it has been demonstrated that the ORF3a viral protein of SARS-CoV activates the NLRP3 inflammasome through ubiquitination.

Since agents which prevent NLRP3 activation are known, anti-inflammatory treatments are promising. However, the use of anti-inflammatory medications with immunosuppressive properties such as glucocorticoids and non-steroidal anti-inflammatory drugs (NSAIDs) are not recommendable to combat an infectious disease. Therefore, the identification of molecules which combine anti-inflammatory effects with immunostimulating actions, particularly on the adaptive immune response, is highly desirable. An agent having both these properties is melatonin (Tan DX, Hardeland R (2020). Potential utility of melatonin in deadly infectious diseases related to the overreaction of innate immune response and destructive inflammation: focus on COVID-19. Melatonin Research. 3, 120-143. DOl.org/10.32794/mr11250052).

In COVID-19 infection, free radical formation and massive inflammatory reaction are associated with the release of a large amount of cytokines ("cytokine storm"). The pathogens perse serve as PAMPs (pathogen-associated molecular patterns), molecular patterns which trigger a primary "cytokine storm", whereas damaged host cell molecules work as DAMPs (dangerous-associated molecular patterns), molecular patterns responsible for a secondary "cytokine storm". This becomes a vicious cycle and, if not interrupted, would cause significant tissue and organ damage and possibly death.

Most of the mentioned viral diseases are self-limited by adaptive immune response which depends on cell proliferation, and therefore requires several weeks to develop. Patients are vulnerable and mortality is high in this window period. Controlling innate immune response and reducing inflammation during this period increase patients' tolerance and decreases mortality in fatal virus infection.

Melatonin is a molecule which is capable of breaking this vicious cycle. Melatonin is a free radical scavenger powerful in reducing oxidative tissue damage and also an anti-inflammatory agent effective in curbing "cytokine storms" (Garcia JA, Volt H, Venegas C, Doerrier C, Escames G, Lopez LC, Acuna-Castroviejo D. Disruption of the NF-kappa B/NLRP3 connection by melatonin requires retinoid-related orphan receptor-alpha and blocks the septic response in mice. FASEB J 2015;29:3863-3875).

As a result, melatonin can increase host tolerance to pathogens and save precious time so that patients develop adaptive immune response and eventually recover from pathogen attack. Furthermore, melatonin also enhances adaptive immune response by increasing T-lymphocyte and B-cell proliferation to generate specific antibodies (Ramos A, Miguez MP, Morgado S, Sanchez-Correa B, Gordillo JJ, Casado JG, Tarazona R, Regodón 1. Melatonin enhances responsiveness to Dichelobacter nodosus vaccine in sheep and increases peripheral blood CD4 T lymphocytes and IgG-expressing B lymphocytes. Vet Immunopathol 2018; 206: doi: 10.1016/j.vetimm.2018.11.006). Various studies have demonstrated the beneficial effects of melatonin in fatal virus infections in different animal models and its therapeutic efficacy in patients with septic shock.

Cathepsins, which are proteins with proteolytic activity, play a very important role in the Covid-19 cell infection process, in addition to inflammatory and pro-oxidative processes. These proteases are essential for virus entry into cell. The virus binds to the ACE2 receptor of the cell, and by means of cathepsin activation, the formation of endosome and its entry into cell are facilitated. It has been demonstrated that melatonin inhibits cathepsins, inhibiting virus proliferation (Yang SF, Chen YS, Chien HW, Wang K, Lin CL, Chiou HL, Lee CY, Chen PN, Hsieh YH. Melatonin attenuates epidermal growth factor-induced cathepsin S expression in ARPE-19 cells: Implications for proliferative vitreoretinopathy. J Pineal Res 2020;68:e12615).

### BRIEF DESCRIPTION OF THE FIGURES

**Figure 1****.** The graph shows an evaluation of whether treatment with intravenous melatonin (according to Example 5) reduces the duration of mechanical ventilation (MV). Likewise, whether treatment with intravenous melatonin is associated with an increase in MV-free days is shown.
**Figure 2****.** The graph shows the mean concentration of D-dimeron days 1, 3, 7, 14, 21, and 28 (means) of the participants of Example 5 from the start of the test (% of day 0).
**Figure 3****.** The graph shows the mean concentration of ferrite on days 1, 3, 7, 14, 21, and 28 (means) of the participants of Example 5 from the start of the test (% of day 0).
**Figure 4****.** The graph shows the mean concentration of CRP (C-reactive protein) on days 1, 3, 7, 14, 21, and 28 (means) of the participants of Example 5 from the start of the test (% of day 0).
**Figure 5****.** The graph shows the mean concentration of IL6 on days 1, 3, 7, 14, 21, and 28 (means) of the participants of Example 5 from the start of the test (% of day 0).
**Figure 6****.** The graph shows the mean concentration of lymphocytes on days 1, 3, 7, 14, 21, and 28 (means) of the participants of Example 5 from the start of the test (% of day 0).
**Figure 7****.** The graph shows the mean hospital stay of the participants of Example 5. The duration of hospital admission is significantly shorter in patients treated with melatonin than in patients treated with placebo. There is a 15-day reduction in hospital stay for patients treated with melatonin compared to placebo.

### DETAILED DESCRIPTION OF THE INVENTION

The authors of the present invention have developed an aqueous melatonin composition with unexpected long-term stability **that allows high concentrations of this water-insoluble active substance** (melatonin). The properties of this composition make it useful as an injectable, for example, for intravenous administration at high concentrations of melatonin. In this sense, the present invention relates to the use of said aqueous melatonin composition for use thereof in the treatment of viral infections such as SARS-CoV-2, preferably at a concentration of 6 mg of melatonin per mL, more preferably for parenteral administration thereof, preferably through intravenous perfusion by means of drip.

It is important to point out that, in the present invention, the anti-inflammatory and antioxidant effects of melatonin for the treatment of viral infections such as SARS-CoV-2 are exerted at high doses.

Specifically, the doses considered in the present invention for humans are administered at minimum doses of 0.81 mg/kg/day, preferably between 0.81 mg/kg/day and 20 mg/kg/day, more preferably between 0.81 mg/kg/day and 15 mg/kg/day, more preferably between 0.81 mg/kg/day and 10 mg/kg/day, more preferably between 0.81 mg/kg/day and 9.80 mg/kg/day, more preferably between 0.81 mg/kg/day and 8.10 mg/kg/day, even more preferably about 5 mg/kg/day, more particularly about 5 mg/kg/day of intravenous melatonin every 6 hours. In terms of absolute amounts and considering a weight of about 80 kg as standard weight, the doses considered would range from 66 mg/day to 1600 mg/day, preferably from 150 to 750 mg/day, more particularly from 150 to 250 mg/day.

In a preferred embodiment, the doses of melatonin considered in the present invention for humans, through the parenteral route (specifically intravenous perfusion, by means of dripping), would range from 150 to 750 mg/day, more particularly from 150 to 500 mg/day, even more specifically, from about 180 to 480 mg/day.

In a preferred embodiment, for those subjects with serious viral infections who are usually bedridden in ICU and often require the use of a respirator, the daily doses depend on the weight of the patient and range from about 2 to 8 mg/kg/day (the dose will be adjusted depending on severity), preferably about 5 mg/kg/day, more particularly about 5 mg/kg/day of intravenous melatonin every 6 hours. Other possibilities are established as follows:
- patients with a weight less than 80 kg (weight < 80 kg): 240 mg/day, even more preferably with a dosage regimen of 60 mg in 4 doses administered every 6 h, and
- patients with a weight more than or equal to 80 kg (weight ≥ 80 kg): 480 mg/day, even more preferably with a dosage regimen of 120 mg in 4 doses administered every 6 h.

In another preferred embodiment, for those subjects without serious viral infections, the dose is about 180 mg/day, administered even more preferably with a regimen of three 60 mg doses every 8 h.

Therefore, a first aspect of the invention relates to a composition (hereinafter "injectable composition of the invention"), suitable for combining with water or a saline solution and for preparing an injectable melatonin composition, comprising propylene glycol, polyethylene glycol, and melatonin or a derivative, a salt, a prodrug, or a solvate thereof, for use thereof in the treatment of inflammatory response in a human subject affected by a viral infection, wherein said composition is administered parenterally, preferably through the intravenous route, more preferably in a drip, at minimum doses of 0.81 mg/kg/day, preferably between 0.81 mg/kg/day and 20 mg/kg/day, more preferably between 0.81 mg/kg/day and 15 mg/kg/day, more preferably between 0.81 mg/kg/day and 10 mg/kg/day, more preferably between 0.81 mg/kg/day and 9.80 mg/kg/day, more preferably between 0.81 mg/kg/day and 8.10 mg/kg/day, even more preferably about 5 mg/kg/day, more particularly about 5 mg/kg/day of intravenous melatonin every 6 hours. In terms of absolute amounts and considering a weight of about 80 kg as standard weight, the doses considered would range from 66 mg/day to 1600 mg/day, preferably from 150 to 750 mg/day, more particularly from 150 to 250 mg/day. Preferably, for those subjects with serious viral infections who are usually bedridden in ICU and often require the use of a respirator, the daily doses depend on the weight of the patient and range from about 2 to 8 mg/kg/day (the dose will be adjusted depending on severity), preferably about 5 mg/kg/day, more particularly about 5 mg/kg/day of intravenous melatonin every 6 hours, or specifically:
- patients with a weight less than 80 kg (weight < 80 kg): 240 mg/day, even more preferably with a dosage regimen of 60 mg in 4 doses administered every 6 h, and
- patients with a weight more than or equal to 80 kg (weight ≥ 80 kg): 480 mg/day, even more preferably with a dosage regimen of 120 mg in 4 doses administered every 6 h.

In another preferred embodiment, for those subjects without serious viral infections, the dose is about 180 mg/day, administered even more preferably with a regimen of three 60 mg doses every 8 h.

In a preferred embodiment of the first aspect of the invention, the composition is used for the treatment of viral infections such as those caused by SARS-CoV, SARS-CoV-2, MERS, Ebola, as well as infections caused by other related viruses (such as those caused by coronavirus). Preferably, the composition is used for reducing the inflammatory response and oxidative damage to the viral infection, giving rise to reduced morbimortality in these patients. Preferably, the composition is used for the treatment of SARS-CoV-2, and allows reducing the inflammatory response and oxidative damage, and/or the virus entry into cell, giving rise to a reduced morbimortality in these patients.

In a preferred embodiment of the first aspect of the invention, the composition is administered at a concentration of about 6 mg of melatonin per mL.

In another preferred embodiment of the first aspect of the invention, the composition is lyophilized (hereinafter "lyophilized composition of the invention") and comprises a suitable proportion of each of the following components: propylene glycol, polyethylene glycol, and melatonin or a derivative, a salt, a prodrug, or a solvate thereof, in such a way that, once rehydrated, the injectable composition of the invention can be obtained.

It should be mentioned that, in the context of the present invention, both the injectable composition of the invention and the lyophilized composition of the invention are jointly referred to as "composition of the invention".

In another preferred embodiment of the first aspect of the invention, the composition is in the form of a pharmaceutically acceptable injectable solution comprising water or a saline solution, propylene glycol, polyethylene glycol, and melatonin or a derivative, a salt, a prodrug, or a solvate thereof; wherein the composition or injectable solution comprises:
- between 5 and 50 grams for every 100 ml of the total solution (w/v) of propylene glycol, preferably between 10 and 30 grams for every 100 ml of the total solution (w/v), more preferably about 20 grams for every 100 ml of the total solution (w/v);
- between 5 and 50 grams for every 100 ml of the total solution (w/v) of polyethylene glycol, preferably between 20 and 40 grams for every 100 ml of the total solution (w/v), more preferably about 30 grams for every 100 ml of the total solution (w/v);
- between 40 and 80 milligrams for every 10 ml of the total solution (w/v) of melatonin, preferably between 50 and 70 milligrams for every 10 ml of the total solution (w/v), more preferably between 55 and 65 milligrams for every 10 ml of the total solution (w/v), more preferably between 58 and 62 milligrams for every 10 ml of the total solution (w/v), more preferably between 59 and 61 milligrams for every 10 ml of the total solution (w/v), more preferably about 0.6 g/100 ml of the total solution; and
- a sufficient amount of water or saline solution.

Therefore, the composition which has been described allows high loads of melatonin required for use thereof as an antiviral treatment, while being stable at the same time, since it has been found that melatonin is significantly solubilized at the relatively low concentrations of propylene glycol (PPG) used in the present invention, thereby reducing the risk of irritation or pain which may accompany the administration of high concentrations of PPG as a side effect. High doses of melatonin can therefore be administered without administering at the same time large amounts of propylene glycol which, at very high doses, may have toxic effects, and in any case the risk of side effects is reduced.

The composition of the invention (both the injectable composition and the lyophilized composition) may also optionally comprise other pharmaceutically acceptable excipients. According to the definition of the EMEA, excipient is any component in the composition other than the active substance.

Examples of excipients which can be used in the composition include antimicrobial preservatives, such as methylparaben, propylparaben; antioxidants, such as sodium metabisulfite, propyl gallate; stabilizing and suspending agents, such as soluble or inflatable modified celluloses, for example, sodium carboxymethylcellulose (Aquasorb, Blanose, Nymcel); tonicity agents, such as sodium chloride; or solubilizers, such as propylene glycol or polyethylene glycols. These excipients must be found within the limits of the definition of the invention.

According to the present invention, a "*pharmaceutically acceptable*" composition or component thereof indicates that composition or component is physiologically tolerable and the administration of which entails a low risk of allergies, side effects, adverse events, or other similar reactions, such as gastric disorders, dizziness, and the like, when administered to a human being. Preferably, as it is used herein, the expression "*pharmaceutically acceptable*" means that it has been approved by a regulatory agency of the state or federal government or is listed in the United States Pharmacopeia or another generally recognized pharmacopeia for use thereof in animals and more particularly in human beings. Therefore, the composition of the invention is free of pyrogens.

The composition of the invention includes melatonin, as well as a derivative, a salt, a prodrug, or a solvate thereof. For example, pharmaceutically acceptable salts are synthesized from melatonin by means of conventional chemical methods, generally by reacting it with a suitable acid in water or in an organic solvent or in a mixture of the two. Generally, non-aqueous means such as ether, ethyl acetate, ethanol, isopropanol, or acetonitrile are preferred. Examples of the acid addition salts include mineral acid addition salts such as, for example, hydrochloride, hydrobromide, hydroiodide, sulfate, nitrate, phosphate, and organic acid addition salts such as, for example, acetate, maleate, fumarate, citrate, oxalate, succinate, tartrate, malate, mandelate, methanesulfonate, and p-toluenesulfonate.

The term "*prodrug*"*,* as it is used in this application, is defined herein to mean a chemical compound that has undergone a chemical derivation such as a substitution or addition of an additional chemical group in order to change (for pharmaceutical use) any of its physicochemical properties, such as solubility or bioavailability, for example, the ester, ether, or amide derivatives of an active compound which provide the active compound itself upon administration to a subject. Examples of well-known methods for producing a prodrug of a given active compound are known to those skilled in the art and can be found, for example, in Krogsgaard-Larsen et al., Textbook of Drug Design and Discovery, Taylor & Francis (April 2002).

Particularly favored prodrugs are those which increase the bioavailability of the compounds of this invention when such compounds are administered to a patient (for example, allowing an orally administered compound to be more readily absorbed in the blood) or which improve the supply of the original compound to a biological compartment (for example, the brain or lymphatic system) with respect to the original species.

The term "*solvate*" according to this invention shall be understood to mean any form of melatonin according to the invention having another molecule (most probably a polar solvent) attached by means of a non-covalent bond. Examples of such solvates include hydrates and alcoholates, for example, methanolates. The preparation of salts, solvates, and prodrugs can be carried out by means of methods known in the art. It should be noted that non-pharmaceutically acceptable salts, solvates, or prodrugs also fall within the scope of the invention given that they may be useful in the preparation of pharmaceutically acceptable salts, solvates, or prodrugs.

Various melatonin derivatives, which are also included in the present invention, are known in the state of the art. According to a particular embodiment, the melatonin derivative is defined according to formula (I), a salt, a prodrug, or a solvate thereof 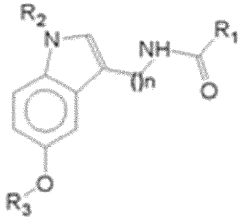 wherein,
n is an integer selected from the group consisting of 1, 2, 3, and 4;
R1 and R3 are independently selected from the group consisting of linear or branched C1-C4 alkyl; and
R2 is selected from the group consisting of hydrogen, linear or branched C1-C4 alkyl, -C(=O)O-Ra, and -C(=O)-N(H)-Ra, wherein Ra is a linear or branched C1-C4 alkyl group.

In a particular embodiment, the injectable composition of the invention is injectable by intravenous route. A particular aspect includes the presence of a second medication in the composition of the invention. Said second medication can be part of the composition or can be provided as a separate composition for administration at the same time or at different times.

As mentioned, the present invention relates to the use of melatonin, its salts, prodrugs, derivatives, or solvates as an antiviral treatment. In a particular embodiment, said use involves the minimum administration of 0.81 mg/kg/day, preferably between 0.81 mg/kg/day and 20 mg/kg/day, more preferably between 0.81 mg/kg/day and 15 mg/kg/day, more preferably between 0.81 mg/kg/day and 10 mg/kg/day, more preferably between 0.81 mg/kg/day and 9.80 mg/kg/day, more preferably between 0.81 mg/kg/day and 8.10 mg/kg/day. In terms of absolute amounts and considering a weight of about 80 kg as standard weight, the doses considered would range from 66 mg/day to 1600 mg/day, preferably from 150 to 750 mg/day, more particularly from 150 to 250 mg/day. In another particular embodiment, the amount of melatonin administered through the injectable composition of the invention to a patient is comprised between 30 and 90 mg every 4, 6, or 8 hours, preferably between 40 and 70 mg, more preferably 60 mg, every 4, 6, or 8 hours. More preferably, for patients with serious viral infections who often require a respirator, a 60 mg/10 mL injectable melatonin solution is administered endovenously in a drip at a dose of 60 mg/6 h (10 mL of the solution/6 h), i.e., a total of 240 mg melatonin/day for those patients with a weight < 80 kg 80 kg, and 480 mg melatonin/day for those patients with a weight ≥ 80 kg. The treatment period will last from diagnosis and hospital admission until discharge. More preferably, for another type of mild or moderate patients, a 60 mg/10 mL injectable melatonin solution is administered endovenously in a drip at a dose of 60 mg/8 h (10 mL of the solution/8 h), i.e., a total of 180 mg melatonin/day. The treatment period will last from diagnosis and hospital admission until discharge.

In a particular embodiment, the administration is performed by perfusion. In another embodiment, melatonin, its salts, prodrugs, derivatives, or solvates, is administered 1, 2, 3, 4, 5, or 6 or more times a day until reaching the total daily dose required. The treatment period may vary depending on the patient's evolution, and it usually lasts between 1 and 30 days.

The term "*treatment*" or "*treat*" in the context of this document refers to the administration of a compound or a formulation according to the invention to prevent, improve, or eliminate the disease or one or more symptoms associated with said disease. "*Treatment*" also encompasses the prevention, improvement, or elimination of physiological sequelae of the disease.

Throughout the description and claims, the word "*comprises*" and variants thereof do not seek to exclude other technical features, additives, components, or steps. For those skilled in the art, other objects, advantages, and features of the invention will be inferred in part from the description and in part from the practice of the invention. In a particular embodiment, the word "*comprises*" can be interpreted as "*consists of*"*.*

The following examples and drawings are provided by way of illustration and do not seek to limit the present invention.

### EXAMPLES

The invention will be illustrated below by means of tests performed by the inventors, clearly showing the stability and effectiveness of the composition of the invention.

### Example 1. Preparation of the composition of the invention

Melatonin for the injectable solution has been prepared at a concentration of 6 mg/ml in about 20% propylene glycol and about 30% polyethylene glycol and with pyrogen-free water in sufficient amount (API).

**Table 1. Qualitative and quantitative composition of the tested composition**

| **Component** | **Composition (per mL)** | **Function** |
|---|---|---|
| Melatonin | 6.0 mg | Active substance |
| Propylene glycol | 200.0 mg | Excipient |
| Polyethylene glycol (macrogol) | 300.0 mg | Excipient |
| Water for injectables | q.s. 1 ml | Solvent |

The material used for packaging the composition described in Table 1 were type I glass vials (EP) previously sterilized in an oven.

### Example 2. Stability tests

### 2.1. General principles

The present study is a stability study of the product specified in Table I from preparation and throughout a 6-month period. To that end, three industrial sized baths each of them comprising 1000 vials of the product specified in Table I have been used.

### 2.2 Stability test results.

**Table 2: Long-term stability data containing the 6 mg/ml solution described in Table I.**

| **6 mg/ml injectable melatonin solution in vials.** | | | | |
|---|---|---|---|---|
| **Batch size:** 1,000 vials | | | **Bath type:** Scaled-down batch | |
| **Date of manufacture:** | 27/02/2013 | | **Container for the product once closed:** Type I glass vials containing a clear, colorless, particle-free solution. | |
| **Test conditions:** | Temperature 25°C+/- 2°C | | | |
| | Relative humidity 60^{a} +/- 5% | | | |
| **Product position:** | right | | | |

| **Parameter** | **Technical specifications** | **0** | **3** | **6** |
|---|---|---|---|---|
| | | **(2013/03)** | **(2013/06)** | **(2013/06)** |
| Product appearance | Glass vial containing a clear, colorless solution. Particle-free. | Complies with technical specification | Complies with technical specification | Complies with technical specification |
| Melatonin identification (UV-visible) | Positive | Positive | Positive | Positive |
| pH determination | 55-7.5 | 6.6 | 6.6 | 6.5 |
| Density | 1.04-1.08 | 1.07 | 1.06 | 1.06 |
| Extractable volume | More than or equal to 9 mL | 9.5 | 9.4 | 9.3 |
| Degradation products | | | | |
| Total | < or = 1.5% | n.d. | n.d. | 0.11 |
| 5-methoxytryptamine | < or = 0.50% | n.d. | n.d. | n.d. |
| Unknown individuals | < or =0.10% | n.d. | n.d. | |
| TRR0.7 | < or =0.10% | | | 0.01 |
| TRR2.0 | < or =0.10% | | | 0.08 |
| Melatonin content | 5.7-6.5 mg/ml | 5.96 | 5.84 | 5.76 |
| Subvisible particles | > = 10 um: < or =6,000 | 115 | 135 | 108 |
| | particles/vial > = 25 um: < or =600 particles/vial | 21 | 25 | 18 |
| Leak-tightness | Leak-tight vial | Complies with technical specification | Complies with technical specification | Complies with technical specification |
| Bacterial endotoxins (LAL test) | < or = 35.1 EU/ml | <35.1 | - | - |
| Sterility test | Sterile | Sterile | - | - |

It can be concluded from the long-term stability test shown that, the described vials containing 6 mg/ml injectable melatonin solution comply with the technical specifications required for a product with these characteristics after storage for a period of 6 months.

### Example 3. Clinical study with septic patients.

The composition of the invention, i.e., the 6 mg/ml injectable melatonin solution in vials described in Example 1, hereinafter *"injectable melatonin",* was used in a clinical study with 14 septic patients who have undergone abdominal surgery randomly distributed into 2 study groups (A and B). Group A corresponds to patients which, in addition to common treatment, have received the injectable melatonin at a dose of 60 mg/day for 5 days, with blood samples being obtained daily to perform successive analytical determinations. Treatment group B has received common treatment and placebo, where the placebo is the same vial with the same excipients, but without the active substance melatonin, blood samples are also obtained daily from each patient of this group to perform successive analytical determinations. The blood samples are named T0, T1, T2, T3, T4, and T5.

The following blood parameters from these samples were analyzed for each of the participating patients: the number of leukocytes, the number of red blood cells, hemoglobin, hematocrit, percentage of neutrophils, percentage of lymphocytes, and the number of platelets. The biochemical parameters determined in each patient participating in the study were: transaminases (GOT and GPT), gamma-glutamyl transferase, creatinine, urea, alkaline phosphatase (ALP), and lactate dehydrogenase (LDH).

### Justification of the determinations performed

Blood parameters consisting of the number of leukocytes, neutrophils, and lymphocytes, as well as the number of platelets, are parameters indicating septic state. In this sense, it is known that sepsis causes a decrease in the percentage of lymphocytes.

The determined biochemical parameters are related to liver function such as:
- Glutamate oxalacetate transaminase or aspartate aminotransferase (GOT/AST): an enzyme belonging to the group of transaminases which, by means of amino group transfer, catalyzes the conversion of amino acids to the corresponding α-oxoacids and vice versa. It is found in the cytoplasm and mitochondria. It is highly specific to liver pathology.
- Glutamate pyruvate transaminase or alanine aminotransferase (GPT/ALT): an enzyme also belonging to the group of transaminases which, by means of amino group transfer, catalyzes the conversion of amino acids to the corresponding α-oxoacids and vice versa. It is recorded with the highest activity in the liver.
- Gamma-glutamyl transferase (GGT) which contributes to the diagnosis and control of hepatobiliary diseases. The enzymatic activity of the GGT is often the only parameter which increases in diseases of this type and has a high sensitivity.
- Alkaline phosphatase (ALP/FA): four structural genotypes are found in human serum: the liver-bone-kidney type, the intestinal type, the placental type, and the variant of germ cells. This enzyme is found in osteoblasts, hepatocytes, the kidneys, spleen, placenta, prostate, leukocytes, and in the small intestine. The liver-bone-kidney type is of particular importance.

The following parameters related to kidney function have been determined:
- Creatinine is a waste molecule generated from muscle metabolism. Creatinine originates from creatine, a very important molecule for energy production in the muscle. About 2% of creatine in the body is converted into creatinine every day. Creatinine is transported from muscles to the kidney by blood. The kidneys filter most of the creatinine and eliminate it in the urine. Determination of serum creatinine is a test which provides a fairly reliable indication of the state of kidney function.
- Urea, the determination of which is the most widely used test to assess kidney function. Urea is the end product of protein and amino acid metabolism. In protein degradation, proteins are split into amino acids and deaminated. Urea is synthesized in the liver with the ammonia that is formed. This is the most important pathway for degrading excess nitrogen in the human body.

The enzyme lactate dehydrogenase (or also called "lactic acid dehydrogenase" (LDH)), an enzyme which is found in almost all the tissues in the human body, has been determined to detect injuries in tissues such as the liver. It plays an important role in cellular respiration (the process in which glucose from foods is converted into energy which can be used by cells).

Although LDH is abundant in tissue cells, the levels in blood are generally low. However, when tissues are damaged as a result of an injury or a disease, they release more LDH into the blood stream. This increase in the amount of LDH in the blood stream is often caused by the following conditions: liver diseases, heart attacks, anemia, muscle trauma, bone fracture, cancer, infections such as meningitis, encephalitis, or HIV.

### Results and discussion

Data from the study has been statistically analyzed by an independent statistician with the following results being obtained:
- Leukocytes: The results show a drop in the levels of leukocytes in both group A and group B. Although the values of leukocytes drop, on average, in both groups, the differences are not statistically significant.

- Red blood cells: Although the values of red blood cells drop, on average, in both groups, the differences are not statistically significant.
- Hemoglobin: Although the values of hemoglobin drop, on average, in both groups, the differences are not statistically significant.
- Hematocrit: Although the values of hematocrit drop, on average, in both groups, the differences are not statistically significant.
- Platelets: Although the values of platelets increase, on average, in both groups, the differences are not statistically significant.
- Lymphocytes: There are significant differences between groups (p=0.015), i.e., the lymphocyte average is higher in group A (treated with the injectable melatonin) than in group B (placebo), regardless of time (the differences are the same in all the times measured). Furthermore, the effect of time is statistically significant (p=0.005, because sphericity is not complied with), which means that the increase in the levels of lymphocytes is different in the different time points measured. Specifically, the differences are due to times 4 and 5 with respect to the initial time.
- Neutrophils: Like in the case of lymphocytes, there are significant differences between the study groups (p=0.007), i.e., the neutrophil average is higher in group B (placebo) than in group A (melatonin), regardless of time (the differences are the same in all the times measured). The differences in time are statistically significant (p=0.042, because sphericity is not complied with). Practically, it can be said that the drop starts to be significant from time 3 in group A (melatonin).
- GOT: Despite the initial levels of GOT being higher in patients subjected to treatment (group A), differences in average values are not statistically significant (p=0.633). The change in different time points is not significant either, i.e., the levels of GOT remain virtually the same.
- GPT: The change in the levels of GPT is not significant in the different times measured. The average values are not statistically significant either.
- GGT: The change in the levels of GGT is not significant in the different times measured. The average values are not statistically significant either.
- ALP (alkaline phosphatase): The change in alkaline phosphatase is not significant in the different times measured. The average values are not statistically significant either.
- Urea: There are no significant differences in the levels of urea over time, said levels remaining virtually the same in both groups (Friedman test, p=0.205 for group A and p=0.959 for group B). If the levels of urea between the two groups are compared in each measured time point, it is observed that the levels of urea are higher in group B (placebo) in all the times measured (with the exception of the last). The differences are statistically significant.
- Creatinine: There are no significant differences in the levels of creatinine over time, said levels remaining the same in both groups (Friedman test, p=0.122 for group A and p=0.831 for group B). If the levels of creatinine between the two groups are compared in each measured time point, it is observed that there are no statistically significant differences in the levels of creatinine between groups A and B.
- LDH: There are no significant differences in the levels of LDH overtime, said levels remaining virtually the same in both groups (Friedman test, p=0.355 for group A and p=0.921 for group B). If the levels of LDH between the two groups are compared in each measured time point, it is observed that the levels of LDH are higher in group A (melatonin) in all the times measured, although the differences are not statistically significant.

### Analyses with non-parametric tests

Although, despite the small sample size, the hypothesis of normality of all the variables is fulfilled, non-parametric tests have been used because they are more robust and suitable when samples are so small.

Taking this into account, on one hand, the time evolution of the different parameters in each group have been compared separately using the Friedman test for independent samples. Statistically significant differences (p<0.10) are obtained in the following parameters for time evolution:
- The level of hemoglobin observed in different times for group A (p=0.069).
- The level of platelets observed in different times for group A (p=0.056) and group B (p=0.069). The level of lymphocytes observed in different times for group A (p=0.030) and group B (p=0.085).

- The level of neutrophils observed in different times for group A (p=0.070).
- The level of GOT observed in different times for group A (p=0.013) and group B (p=0.077).
- The level of GPT observed in different times for group B (p=0.004).
- The level of GGT observed in different times for group A (p=0.079).

On the other hand, direct differences between groups A and B in each time point have been compared independently using Mann-Whitney test for independent samples. The results were as follows:
- There are differences between A and B in the levels of red blood cells (p=0.026), hemoglobin (p=0.038), and hematocrit (p=0.053) in time T2.
- There are differences in the levels of lymphocytes in all the times.
- There are differences between A and B in the levels of neutrophils in T2 (p=0.007) and T3 (p=0.011).
- There are differences between A and B in the levels of GPT in T5 (p=0.038).

### Conclusions

Treatment with the injectable melatonin of septic patients of group A who receive the injectable shows a gradual increase in the percentage of lymphocytes. This increase is statistically significant. Similarly, these patients show a statistically significant drop in the percentage of neutrophils. Both the increase in lymphocytes and the drop in neutrophils occur in all the times of the study, with levels close to normal values in healthy individuals being achieved at the end of the study period. This situation represents an immunological recovery in patients who receive treatment with the injectable melatonin, as the balance between lymphocytes and neutrophils in patients who receive treatment with the injectable is in equilibrium.

On the other hand, treatment with the injectable melatonin does not entail liver or kidney damage in patients who receive treatment with the injectable.

### Details of the statistical analysis

Details of the analysis performed on the parameters measured in all the times (T0, ..., T5) of the study are set forth below.

### Leukocytes

| | **Group** | **Mean** | **Standard deviation** | **N** |
|---|---|---|---|---|
| Leukocytes | A | 14.7329 | 9.09637 | 7 |
| 10_3 ml | B | 19.2857 | 7.82250 | 7 |
| T0 | Total | 17.0093 | 8.48601 | 14 |
| Leukocytes | A | 14.4200 | 9.59540 | 7 |
| 10_3 ml | B | 19.0529 | 8.86479 | 7 |
| T1 | Total | 16.7364 | 9.19473 | 14 |
| Leukocytes | A | 11.6614 | 4.49294 | 7 |
| 10_3 ml | B | 16.6786 | 5.94305 | 7 |
| T2 | Total | 14.1700 | 5.69169 | 14 |
| Leukocytes | A | 10.1457 | 4.71706 | 7 |
| 10_3 ml | B | 15.5086 | 5.02559 | 7 |
| T3 | Total | 12.8271 | 5.44697 | 14 |
| Leukocytes | A | 11.9529 | 8.60995 | 7 |
| 10_3 ml | B | 15.4971 | 3.56829 | 7 |
| T4 | Total | 13.7250 | 6.59341 | 14 |
| Leukocytes | A | 12.1114 | 6.91874 | 7 |
| 10_3 ml | B | 12.8900 | 2.85623 | 7 |
| T5 | Total | 12.5007 | 5.10116 | 14 |

Although the values of leukocytes drop, on average, in both groups, the differences are not statistically significant.

### Red blood cells

| | **Group** | **Mean** | **Standard deviation** | **N** |
|---|---|---|---|---|
| Red blood cells | A | 4.1243 | 0.83320 | 7 |
| 10_3 ml | B | 3.4043 | 1.06878 | 7 |
| T0 | Total | 3.7643 | 0.99358 | 14 |
| Red blood cells | A | 3.8457 | 0.62612 | 7 |
| 10_3 ml | B | 3.7671 | 0.74904 | 7 |
| T1 | Total | 3.8064 | 0.66449 | 14 |
| Red blood cells | A | 3.8100 | 0.32655 | 7 |
| 10_3 ml | B | 3.1486 | 0.64300 | 7 |
| T2 | Total | 3.4793 | 0.59818 | 14 |
| Red blood cells | A | 3.6071 | 0.31700 | 7 |
| 10_3 ml | B | 3.2843 | 0.49315 | 7 |
| T3 | Total | 3.4457 | 0.43207 | 14 |
| Red blood cells | A | 3.6371 | 0.52506 | 7 |
| 10_3 ml | B | 3.4357 | 0.56151 | 7 |
| T4 | Total | 3.5364 | 0.53262 | 14 |
| Red blood cells | A | 3.6900 | 0.52173 | 7 |
| 10_3 ml | B | 3.2643 | 0.66795 | 7 |
| T5 | Total | 3.4771 | 0.61672 | 14 |

Although the values of red blood cells drop, on average, in both groups, the differences are not statistically significant.

### Hemoglobin

| | **Group** | **Mean** | **Standard deviation** | **N** |
|---|---|---|---|---|
| Hemoglobin 10_3 ml T0 | A | 12.0714 | 2.59597 | 7 |
| | B | 10.1143 | 2.72484 | 7 |
| | Total | 11.0929 | 2.75107 | 14 |
| Hemoglobin 10_3 ml T1 | A | 11.0857 | 1.94202 | 7 |
| | B | 11.3143 | 1.88275 | 7 |
| | Total | 11.2000 | 1.84140 | 14 |
| Hemoglobin 10_3 ml T2 | A | 11.0571 | .85021 | 7 |
| | B | 9.4143 | 1.63649 | 7 |
| | Total | 10.2357 | 1.51536 | 14 |
| Hemoglobin 10_3 ml T3 | A | 10.6429 | 1.01301 | 7 |
| | B | 9.8143 | 1.15243 | 7 |
| | Total | 10.2286 | 1.12758 | 14 |
| Hemoglobin 10_3 ml T4 | A | 10.6143 | 1.37408 | 7 |
| | B | 10.1429 | 1.28693 | 7 |
| | Total | 10.3786 | 1.30217 | 14 |
| Hemoglobin 10_3 ml T5 Total | A | 10.7286 | 1.52065 | 7 |
| | B | 9.5143 | 1.54103 | 7 |
| | | 10.1214 | 1.60009 | 14 |

Although the values of hemoglobin drop, on average, in both groups, the differences are not statistically significant.

### Hematocrit

| | **Group** | **Mean** | **Standard deviation** | **N** |
|---|---|---|---|---|
| Hematocrit (%) T0 | A | 34.2857 | 7.21097 | 7 |
| | B | 29.4714 | 8.19445 | 7 |
| | Total | 31.8786 | 7.82503 | 14 |
| Hematocrit (%) T1 | A | 32.1143 | 5.31583 | 7 |
| | B | 32.9571 | 5.82662 | 7 |
| | Total | 32.5357 | 5.37610 | 14 |
| Hematocrit (%) T2 | A | 31.9714 | 2.38447 | 7 |
| | B | 27.6143 | 5.00447 | 7 |
| | Total | 29.7929 | 4.39256 | 14 |
| Hematocrit (%) T3 | A | 30.3857 | 2.65796 | 7 |
| | B | 28.7143 | 3.62971 | 7 |
| | Total | 29.5500 | 3.17702 | 14 |
| Hematocrit (%) T4 | A | 30.6571 | 4.25435 | 7 |
| | B | 30.0571 | 3.99452 | 7 |
| | Total | 30.3571 | 3.97680 | 14 |
| Hematocrit (%) T5 | A | 31.1143 | 4.22588 | 7 |
| | B | 28.5714 | 5.12826 | 7 |
| | Total | 29.8429 | 4.70331 | 14 |

Although the values of hematocrit drop, on average, in both groups, the differences are not statistically significant.

### Platelets

| | **Group** | **Mean** | **Standard deviation** | **N** |
|---|---|---|---|---|
| Platelets 10_3 ml T0 | A | 249.0000 | 165.65526 | 7 |
| | B | 387.7143 | 215.01451 | 7 |
| | Total | 318.3571 | 197.94778 | 14 |
| Platelets 10_3 ml T1 | A | 234.4286 | 183.64355 | 7 |
| | B | 374.5714 | 229.87958 | 7 |
| | Total | 304.5000 | 212.70375 | 14 |
| Platelets 10_3 ml T2 | A | 240.2857 | 196.55594 | 7 |
| | B | 316.8571 | 194.91146 | 7 |
| | Total | 278.5714 | 192.20771 | 14 |
| Platelets 10_3 ml T3 | A | 256.1429 | 187.80879 | 7 |
| | B | 301.7143 | 213.66930 | 7 |
| | Total | 278.9286 | 194.70469 | 14 |
| Platelets 10_3 ml T4 | A | 317.7143 | 250.15576 | 7 |
| | B | 320.8571 | 201.71880 | 7 |
| | Total | 319.2857 | 218.32313 | 14 |
| Platelets 10_3 ml T5 | A | 380.1429 | 309.16847 | 7 |
| | B | 277.7143 | 220.31017 | 7 |
| | Total | 328.9286 | 263.32941 | 14 |

Although the values of platelets increase, on average, in both groups, the differences are not statistically significant.

### Lymphocytes

| | **Group** | **Mean** | **Standard deviation** | **N** |
|---|---|---|---|---|
| Lymphocytes 10_3 ml T0 | A | 7.9571 | 3.79599 | 7 |
| | B | 4.8286 | 2.78132 | 7 |
| | Total | 6.3929 | 3.58554 | 14 |
| Lymphocytes (%) T1 | A | 8.9571 | 5.23159 | 7 |
| | B | 4.6714 | 1.12207 | 7 |
| | Total | 6.8143 | 4.26125 | 14 |
| Lymphocytes (%) T2 | A | 11.9714 | 5.03545 | 7 |
| | B | 5.6143 | 2.59257 | 7 |
| | Total | 8.7929 | 5.06807 | 14 |
| Lymphocytes (%) T3 | A | 14.3571 | 7.43928 | 7 |
| | B | 5.5714 | 2.36130 | 7 |
| | Total | 9.9643 | 6.99270 | 14 |
| Lymphocytes (%) T4 | A | 16.6143 | 11.13903 | 7 |
| | B | 7.4143 | 3.56718 | 7 |
| | Total | 12.0143 | 9.26971 | 14 |
| Lymphocytes (%) T5 | A | 17.1571 | 9.21590 | 7 |
| | B | 8.2571 | 5.01825 | 7 |
| | Total | 12.7071 | 8.49402 | 14 |

### Tests of inter-subjects effects

### Transformed variable: Average

| **Source** | **Type III sum of squares** | **df** | **Quadratic mean** | **F** | **Sig.** |
|---|---|---|---|---|---|
| Intersection | 7497.630 | 1 | 7497.630 | 62.512 | 0.000 |
| Group | 964.252 | 1 | 964.252 | 8.040 | **0.015** |
| Error | 1439.268 | 12 | 119.939 | | |

There are significant differences between groups (p=0.015), i.e., the lymphocyte average is higher in group A than in group B, regardless of time (the differences are the same in all the times measured).

Furthermore, the effect of time is statistically significant (p=0.005, because sphericity is not complied with), which means that the increase in the levels of lymphocytes is different in the different time points measured.

Specifically, the differences are due to times 4 and 5 with respect to the initial time:

### Tests of intra-subjects effects

| | **Source** | **Type III sum of squares** | **df** | **Quadratic mean** | **F** | **Sig.** |
|---|---|---|---|---|---|---|
| Time | Sphericity assumed | 478.435 | 5 | 95.687 | 6.345 | 0.000 |
| | Greenhouse-Geisser | 478.435 | 2.190 | 218.472 | 6.345 | **0.005** |
| | Huynh-Feldt | 478.435 | 2.921 | 163.772 | 6.345 | 0.002 |
| | Lower bound | 478.435 | 1.000 | 478.435 | 6.345 | 0.027 |
| Time * Group | Sphericity assumed | 119.374 | 5 | 23.875 | 1.583 | 0.179 |
| | Greenhouse-Geisser | 119.374 | 2.190 | 54.511 | 1.583 | 0.223 |
| | Huynh-Feldt | 119.374 | 2.921 | 40.862 | 1.583 | 0.212 |
| | Lower bound | 119.374 | 1.000 | 119.374 | 1.583 | 0.232 |
| Error (time) | Sphericity assumed | 904.861 | 60 | 15.081 | | |
| | Greenhouse-Geisser | 904.861 | 26.279 | 34.433 | | |
| | Huynh-Feldt | 904.861 | 35.056 | 25.812 | | |
| | Lower bound | 904.861 | 12.000 | 75.405 | | |

### Tests of intra-subjects contrasts

| **Source** | **Time** | **Type III sum of squares** | **df** | **Quadratic mean** | **F** | **Sig.** |
|---|---|---|---|---|---|---|
| Time | Level 2 with respect to level 1 | 2.486 | 1 | 2.486 | 0.119 | 0.736 |
| | Level 3 with respect to level 1 | 80.640 | 1 | 80.640 | 4.410 | 0.058 |
| | Level 4 with respect to level 1 | 178.571 | 1 | 178.571 | 4.678 | 0.051 |
| | Level 5 with respect to level 1 | 442.406 | 1 | 442.406 | 6.974 | **0.022** |
| | Level 6 with respect to level 1 | 558.183 | 1 | 558.183 | 8.240 | **0.014** |
| Time * Group | Level 2 with respect to level 1 | 4.686 | 1 | 4.686 | .225 | 0.644 |
| | Level 3 with respect to level 1 | 36.483 | 1 | 36.483 | 1.995 | 0.183 |
| | Level 4 with respect to level 1 | 112.011 | 1 | 112.011 | 2.934 | 0.112 |
| | Level 5 with respect to level 1 | 129.018 | 1 | 129.018 | 2.034 | 0.179 |
| | Level 6 with respect to level 1 | 116.583 | 1 | 116.583 | 1.721 | 0.214 |
| Error (time) | Level 2 with respect to level 1 | 249.977 | 12 | 20.831 | | |
| | Level 3 with respect to level 1 | 219.437 | 12 | 18.286 | | |
| | Level 4 with respect to level 1 | 458.117 | 12 | 38.176 | | |
| | Level 5 with respect to level 1 | 761.286 | 12 | 63.440 | | |
| | Level 6 with respect to level 1 | 812.914 | 12 | 67.743 | | |

### Neutrophils

| | **Group** | **Mean** | **Standard deviation** | **N** |
|---|---|---|---|---|
| Neutrophils (%) | A | 87.1143 | 6.82799 | 7 |
| T0 | B | 92.4286 | 5.35435 | 7 |
| | Total | 89.7714 | 6.50792 | 14 |
| Neutrophils (%) | A | 86.3143 | 8.33175 | 7 |
| T1 | B | 92.2714 | 2.84413 | 7 |
| | Total | 89.2929 | 6.73252 | 14 |
| Neutrophils (%) | A | 81.2143 | 6.01953 | 7 |
| T2 | B | 90.6429 | 4.01325 | 7 |
| | Total | 85.9286 | 6.93480 | 14 |
| Neutrophils (%) | A | 79.0714 | 8.00411 | 7 |
| T3 | B | 91.7571 | 3.77618 | 7 |
| | Total | 85.4143 | 8.91497 | 14 |
| Neutrophils (%) | A | 77.8143 | 12.25988 | 7 |
| T4 | B | 87.7857 | 6.93023 | 7 |
| | Total | 82.8000 | 10.87693 | 14 |
| Neutrophils (%) | A | 76.7286 | 12.21757 | 7 |
| T5 | B | 87.9571 | 6.84930 | 7 |
| | Total | 82.3429 | 11.15752 | 14 |

Like in the preceding case, there are significant differences between groups (p=0.007), i.e., the neutrophil average is higher in group B than in group A, regardless of time (the differences are the same in all the times measured).

### Tests of inter-subjects effects

### Transformed variable: Average

| **Source** | **Type III sum of squares** | **df** | **Quadratic mean** | **F** | **Sig.** |
|---|---|---|---|---|---|
| Intersection | 103363.479 | 1 | 103363.479 | 3804.053 | 0.000 |
| Group | 289.683 | 1 | 289.683 | 10.661 | **0.007** |
| Error | 326.063 | 12 | 27.172 | | |

The differences in time are statistically significant (p=0.042, because sphericity is not complied with). Practically, it can be said that the drop starts to be significant from time 3.

### Tests of intra-subjects effects

| **Source** | | **Type III sum of squares** | **df** | **Quadratic mean** | **F** | **Sig.** |
|---|---|---|---|---|---|---|
| Time | Sphericity assumed | 685.940 | 5 | 137.188 | 3.898 | 0.004 |
| | Greenhouse-Geisser | 685.940 | 1.725 | 397.654 | 3.898 | **0.042** |
| | Huynh-Feldt | 685.940 | 2.156 | 318.204 | 3.898 | **0.030** |
| | Lower bound | 685.940 | 1.000 | 685.940 | 3.898 | **0.072** |
| Time * Group | Sphericity assumed | 148.626 | 5 | 29.725 | 0.845 | 0.524 |
| | Greenhouse-Geisser | 148.626 | 1.725 | 86.162 | 0.845 | 0.428 |
| | Huynh-Feldt | 148.626 | 2.156 | 68.947 | 0.845 | 0.449 |
| | Lower bound | 148.626 | 1.000 | 148.626 | 0.845 | 0.376 |
| Error (time) | Sphericity assumed | 2111.492 | 60 | 35.192 | | |
| | Greenhouse-Geisser | 2111.492 | 20.700 | 102.007 | | |
| | Huynh-Feldt | 2111.492 | 25.868 | 81.626 | | |
| | Lower bound | 2111.492 | 12.000 | 175.958 | | |

### Tests of contrast intra-subjects

| **Source** | **Time** | **Type III sum of squares** | **df** | **Quadratic mean** | **F** | **Sig.** |
|---|---|---|---|---|---|---|
| Time | Level 2 with respect to level 1 | 3.206 | 1 | 3.206 | 0.073 | 0.791 |
| | Level 3 with respect to level 1 | 206.746 | 1 | 206.746 | 14.423 | **0.003** |
| | Level 4 with respect to level 1 | 265.786 | 1 | 265.786 | 3.255 | 0.096 |
| | Level 5 with respect to level 1 | 680.411 | 1 | 680.411 | 4.826 | **0.048** |
| | Level 6 with respect to level 1 | 772.571 | 1 | 772.571 | 4.499 | **0.055** |
| Time * Group | Level 2 with respect to level 1 | 1.446 | 1 | 1.446 | 0.033 | 0.859 |
| | Level 3 with respect to level 1 | 59.246 | 1 | 59.246 | 4.133 | 0.065 |
| | Level 4 with respect to level 1 | 190.183 | 1 | 190.183 | 2.329 | 0.153 |
| | Level 5 with respect to level 1 | 75.911 | 1 | 75.911 | 0.538 | 0.477 |
| | Level 6 with respect to level 1 | 122.426 | 1 | 122.426 | 0.713 | 0.415 |
| Error (time) | Level 2 with respect to level 1 | 525.577 | 12 | 43.798 | | |
| | Level 3 with respect to level 1 | 172.009 | 12 | 14.334 | | |
| | Level 4 with respect to level 1 | 979.811 | 12 | 81.651 | | |
| | Level 5 with respect to level 1 | 1691.737 | 12 | 140.978 | | |
| | Level 6 with respect to level 1 | 2060.663 | 12 | 171.722 | | |

### GOT

| **Group** | | **Mean** | **Standard deviation** | **N** |
|---|---|---|---|---|
| GOT | A | 65.1667 | 51.40201 | 6 |
| T0 | B | 27.9500 | 25.27795 | 6 |
| | Total | 46.5583 | 43.23398 | 12 |
| GOT | A | 56.3333 | 48.38457 | 6 |
| T1 | B | 38.8333 | 22.24785 | 6 |
| | Total | 47.5833 | 37.04901 | 12 |
| GOT | A | 41.0000 | 41.26984 | 6 |
| T2 | B | 45.6667 | 53.67184 | 6 |
| | Total | 43.3333 | 45.71121 | 12 |
| GOT | A | 38.3333 | 34.93232 | 6 |
| T3 | B | 35.0333 | 24.83881 | 6 |
| | Total | 36.6833 | 28.94954 | 12 |
| GOT | A | 38.1667 | 35.92446 | 6 |
| T4 | B | 40.7667 | 33.31556 | 6 |
| | Total | 39.4667 | 33.06020 | 12 |
| GOT | A | 44.8333 | 48.67614 | 6 |
| T5 | B | 35.2500 | 29.48856 | 6 |
| | Total | 40.0417 | 38.69488 | 12 |

Despite the initial levels of GOT being higher in patients with treatment A, differences in average values are not statistically significant (p=0.633). The change in different time points is not significant either, i.e., the levels of GOT remain virtually the same.

### Tests of inter-subjects effects

### Transformed variable: Average

| **Source** | **Type III sum of squares** | **df** | **Quadratic mean** | F | **Sig.** |
|---|---|---|---|---|---|
| Intersection | 21448.926 | 1 | 21448.926 | 17.157 | 0.002 |
| Group | 303.343 | 1 | 303.343 | 0.243 | **0.633** |
| Error | 12501.881 | 10 | 1250.188 | | |

### GPT

| **Group** | | **Mean** | **Standard deviation** | **N** |
|---|---|---|---|---|
| GPT | A | 51.0000 | 54.22791 | 7 |
| T0 | B | 15.7167 | 9.56147 | 6 |
| | Total | 34.7154 | 42.93710 | 13 |
| GPT | A | 45.0000 | 46.54747 | 7 |
| T1 | B | 25.5000 | 18.09696 | 6 |
| | Total | 36.0000 | 36.36161 | 13 |
| GPT | A | 40.2857 | 29.78654 | 7 |
| T2 | B | 24.0000 | 12.69646 | 6 |
| | Total | 32.7692 | 24.12866 | 13 |
| GPT | A | 32.7143 | 21.06114 | 7 |
| T3 | B | 17.7500 | 7.27839 | 6 |
| | Total | 25.8077 | 17.43982 | 13 |
| GPT | A | 30.5714 | 19.26012 | 7 |
| T4 | B | 17.6000 | 7.75113 | 6 |
| | Total | 24.5846 | 15.99405 | 13 |
| GPT | A | 30.2857 | 21.47645 | 7 |
| T5 | B | 13.6667 | 6.59293 | 6 |
| | Total | 22.6154 | 17.97470 | 13 |

The change in the levels of GPT is not significant in the different times measured. The average values are not statistically significant either.

### GGT

| | **Group** | **Mean** | **Standard deviation** | **N** |
|---|---|---|---|---|
| GGT | A | 150.2857 | 158.16206 | 7 |
| T0 | B | 94.9333 | 50.15283 | 6 |
| | Total | 124.7385 | 119.91896 | 13 |
| GGT | A | 131.7143 | 144.03786 | 7 |
| T1 | B | 94.6667 | 53.64202 | 6 |
| | Total | 114.6154 | 109.27911 | 13 |
| GGT | A | 130.2857 | 140.32904 | 7 |
| T2 | B | 82.3333 | 35.61835 | 6 |
| | Total | 108.1538 | 104.85136 | 13 |
| GGT | A | 142.4286 | 160.26526 | 7 |
| T3 | B | 128.0000 | 94.47963 | 6 |
| | Total | 135.7692 | 128.91027 | 13 |
| GGT | A | 147.7143 | 134.40575 | 7 |
| T4 | B | 211.5000 | 186.63735 | 6 |
| | Total | 177.1538 | 156.97709 | 13 |
| GGT | A | 240.8571 | 204.12858 | 7 |
| T5 | B | 194.5000 | 194.53611 | 6 |
| | Total | 219.4615 | 192.82445 | 13 |

The change in the levels of GGT is not significant in the different times measured. The average values are not statistically significant either.

### Alkaline phosphatase

| | **Group** | **Mean** | **Standard deviation** | **N** |
|---|---|---|---|---|
| Phosphatase | A | 116.6667 | 87.96969 | 6 |
| T0 | B | 95.0000 | 22.47665 | 6 |
| | Total | 105.8333 | 62.25145 | 12 |
| Phosphatase | A | 100.5000 | 87.36761 | 6 |
| T1 | B | 84.0000 | 30.02665 | 6 |
| | Total | 92.2500 | 62.87813 | 12 |
| Phosphatase | A | 97.0000 | 54.85618 | 6 |
| T2 | B | 74.8333 | 16.40020 | 6 |
| | Total | 85.9167 | 40.29992 | 12 |
| Phosphatase | A | 106.3333 | 65.98081 | 6 |
| T3 | B | 109.1667 | 70.30339 | 6 |
| | Total | 107.7500 | 65.02045 | 12 |
| Phosphatase | A | 108.8333 | 54.57258 | 6 |
| T4 | B | 159.3333 | 118.81021 | 6 |
| | Total | 134.0833 | 92.00836 | 12 |
| Phosphatase | A | 112.1667 | 43.56336 | 6 |
| T5 | B | 143.8333 | 102.89104 | 6 |
| | Total | 128.0000 | 77.12446 | 12 |

The change in alkaline phosphatase is not significant in the different times measured. The average values are not statistically significant either.

### Analysis with non-parametric tests:

On one hand, the time evolution of the different parameters in each group is compared separately (using the Friedman test for independent samples), statistically significant differences are obtained (p<0.10) in:
• The level of hemoglobin observed in different times for group A (p=0.069):

| **Group** | | | **hemoglobin 10_3 ml T0** | **hemoglobin 10_3 ml T1** | **hemoglobin 10_3 ml T2** | **hemoglobin 10_3 ml T3** | **hemoglobin 10_3 ml T4** | **hemoglobin 10_3 ml T5** |
|---|---|---|---|---|---|---|---|---|
| **A** | N | Valid | 7 | 7 | 7 | 7 | 7 | 7 |
| | | Lost | 0 | 0 | 0 | 0 | 0 | 0 |
| | Mean | | **12.0714** | **11.0857** | **11.0571** | **10.6429** | **10.6143** | **10.7286** |
| | Median | | **12.3000** | **11.4000** | **11.0000** | **10.7000** | **10.3000** | **10.9000** |
| | Std. dev. | | 2.59597 | 1.94202 | .85021 | 1.01301 | 1.37408 | 1.52065 |
| | Min. | | 7.40 | 6.90 | 9.50 | 8.80 | 9.20 | 8.40 |
| | Max. | | 16.10 | 12.90 | 12.30 | 11.50 | 13.30 | 13.00 |
| **B** | N | Valid | 7 | 7 | 7 | 7 | 7 | 7 |
| | | Lost | 0 | 0 | 0 | 0 | 0 | 0 |
| | Mean | | 10.1143 | 11.3143 | 9.4143 | 9.8143 | 10.1429 | 9.5143 |
| | Median | | 9.1000 | 11.1000 | 9.0000 | 9.9000 | 10.0000 | 9.6000 |
| | Std. dev. | | 2.72484 | 1.88275 | 1.63649 | 1.15243 | 1.28693 | 1.54103 |
| | Min. | | 8.40 | 8.60 | 7.60 | 8.70 | 8.80 | 7.40 |
| | Max. | | 16.10 | 14.10 | 12.50 | 11.90 | 12.30 | 11.40 |

• The level of platelets observed in different times for group A (p=0.056) and group B (p=0.069):

| **Group** | | | **platelets 10_3 ml T0** | **platelets 10_3 ml T1** | **platelets 10_3 ml T2** | **platelets 10_3 ml T3** | **platelets 10_3 ml T4** | **platelets 10_3 ml T5** |
|---|---|---|---|---|---|---|---|---|
| A | N | Valid | 7 | 7 | 7 | 7 | 7 | 7 |
| | | Lost | 0 | 0 | 0 | 0 | 0 | 0 |
| | Mean | | 249.0000 | 234.4286 | 240.2857 | 256.1429 | 317.7143 | 380.1429 |
| | Median | | 205.0000 | 147.0000 | 152.0000 | 185.0000 | 220.0000 | 269.0000 |
| | Std. dev. | | 165.65526 | 183.64355 | 196.55594 | 187.80879 | 250.15576 | 309.16847 |
| | Min. | | 87.00 | 44.00 | 33.00 | 33.00 | 59.00 | 97.00 |
| | Max. | | 521.00 | 558.00 | 578.00 | 549.00 | 784.00 | 963.00 |
| B | N | Valid | 7 | 7 | 7 | 7 | 7 | 7 |
| | | Lost | 0 | 0 | 0 | 0 | 0 | 0 |
| | Mean | | 387.7143 | 374.5714 | 316.8571 | 301.7143 | 320.8571 | 277.7143 |
| | Median | | 350.0000 | 243.0000 | 185.0000 | 195.0000 | 267.0000 | 196.0000 |
| | Std. dev. | | 215.01451 | 229.87958 | 194.91146 | 213.66930 | 201.71880 | 220.31017 |
| | Min. | | 163.00 | 155.00 | 149.00 | 67.00 | 61.00 | 37.00 |
| | Max. | | 635.00 | 738.00 | 595.00 | 638.00 | 611.00 | 607.00 |

• The level of lymphocytes observed in different times for group A (p=0.030) and group B (p=0.085):

| **Group** | | | **lymphocytes 10_3 ml T0** | **lym phocytes (%) T1** | **lymphocytes (%) T2** | **lym phocytes (%) T3** | **lymphocytes (%) T4** | **lymphocytes (%) T5** |
|---|---|---|---|---|---|---|---|---|
| A | N | Valid | 7 | 7 | 7 | 7 | 7 | 7 |
| | | Lost | 0 | 0 | 0 | 0 | 0 | 0 |
| | Mean | | 7.9571 | 8.9571 | 11.9714 | 14.3571 | 16.6143 | 17.1571 |
| | Median | | 7.2000 | 8.2000 | 11.0000 | 11.9000 | 12.5000 | 15.3000 |
| | Std. dev. | | 3.79599 | 5.23159 | 5.03545 | 7.43928 | 11.13903 | 9.21590 |
| | Min. | | 3.00 | 2.70 | 4.50 | 2.80 | 2.60 | 5.40 |
| | Max. | | 13.40 | 18.90 | 18.20 | 24.60 | 36.60 | 29.70 |
| | Percentiles | 25 | 4.3000 | 5.2000 | 8.0000 | 10.6000 | 11.5000 | 9.7000 |
| | | 50 | 7.2000 | 8.2000 | 11.0000 | 11.9000 | 12.5000 | 15.3000 |
| | | 75 | 12.1000 | 10.8000 | 17.2000 | 20.6000 | 25.6000 | 27.1000 |
| B | N | Valid | 7 | 7 | 7 | 7 | 7 | 7 |
| | | Lost | 0 | 0 | 0 | 0 | 0 | 0 |
| | Mean | | 4.8286 | 4.6714 | 5.6143 | 5.5714 | 7.4143 | 8.2571 |
| | Median | | 3.9000 | 5.2000 | 4.9000 | 5.4000 | 5.8000 | 7.7000 |
| | Std. dev. | | 2.78132 | 1.12207 | 2.59257 | 2.36130 | 3.56718 | 5.01825 |
| | Min. | | 3.30 | 2.60 | 3.10 | 2.20 | 4.30 | 2.00 |
| | Max. | | 11.10 | 5.70 | 10.30 | 9.40 | 14.10 | 17.90 |
| | Percentiles | 25 | 3.5000 | 3.9000 | 3.8000 | 4.3000 | 4.6000 | 4.5000 |
| | | 50 | 3.9000 | 5.2000 | 4.9000 | 5.4000 | 5.8000 | 7.7000 |
| | | 75 | 4.1000 | 5.6000 | 8.0000 | 7.6000 | 9.9000 | 10.1000 |

• The level of neutrophils observed in different times for group A (p=0.070):

| **Group** | | | **neutrophils (%) T0** | **neutrophils (%) T1** | **neutrophils (%) T2** | **neutrophils (%) T3** | **neutrophils (%) T4** | **neutrophils (%) T5** |
|---|---|---|---|---|---|---|---|---|
| A | N | Valid | 7 | 7 | 7 | 7 | 7 | 7 |
| | | Lost | 0 | 0 | 0 | 0 | 0 | 0 |
| | Mean | | 87.1143 | 86.3143 | 81.2143 | 79.0714 | 77.8143 | 76.7286 |
| | Median | | 88.6000 | 86.9000 | 81.1000 | 75.8000 | 79.5000 | 79.7000 |
| | Std. dev. | | 6.82799 | 8.33175 | 6.01953 | 8.00411 | 12.25988 | 12.21757 |
| | Min. | | 74.00 | 73.30 | 72.90 | 71.30 | 56.80 | 61.90 |
| | Max. | | 95.20 | 95.40 | 91.80 | 92.90 | 94.10 | 91.90 |
| | Percentiles | 25 | 83.1000 | 78.7000 | 77.5000 | 74.1000 | 67.3000 | 65.7000 |
| | | 50 | 88.6000 | 86.9000 | 81.1000 | 75.8000 | 79.5000 | 79.7000 |
| | | 75 | 91.0000 | 94.8000 | 84.5000 | 87.6000 | 85.0000 | 89.6000 |
| B | N | Valid | 7 | 7 | 7 | 7 | 7 | 7 |
| | | Lost | 0 | 0 | 0 | 0 | 0 | 0 |
| | Mean | | 92.4286 | 92.2714 | 90.6429 | 91.7571 | 87.7857 | 87.9571 |
| | Median | | 94.3000 | 92.5000 | 92.2000 | 92.4000 | 88.4000 | 89.9000 |
| | Std. dev. | | 5.35435 | 2.84413 | 4.01325 | 3.77618 | 6.93023 | 6.84930 |
| | Min. | | 80.50 | 87.30 | 83.80 | 84.90 | 77.30 | 76.30 |
| | Max. | | 96.20 | 96.10 | 95.60 | 96.10 | 94.50 | 97.30 |
| | Percentiles | 25 | 92.9000 | 90.4000 | 87.0000 | 89.5000 | 80.5000 | 81.7000 |
| | | 50 | 94.3000 | 92.5000 | 92.2000 | 92.4000 | 88.4000 | 89.9000 |
| | | 75 | 94.9000 | 94.1000 | 93.3000 | 95.3000 | 94.2000 | 90.8000 |

• The level of GOT observed in different times for group A (p=0.013) and group B (p=0.077):

| **Group** | | | **GOT T0** | **GOT T1** | **GOT T2** | **GOT T3** | **GOT T4** | **GOT T5** |
|---|---|---|---|---|---|---|---|---|
| A | N | Valid | 7 | 7 | 7 | 6 | 7 | 7 |
| | | Lost | 0 | 0 | 0 | 1 | 0 | 0 |
| | Mean | | 60.4286 | 54.0000 | 41.7143 | 38.3333 | 36.2857 | 42.0000 |
| | Median | | 36.0000 | 40.0000 | 32.0000 | 22.5000 | 22.0000 | 25.0000 |
| | Std. dev. | | 48.56905 | 44.59821 | 37.72141 | 34.93232 | 33.16984 | 45.06292 |
| | Min. | | 16.00 | 13.00 | 8.00 | 15.00 | 18.00 | 21.00 |
| | Max. | | 147.00 | 145.00 | 122.00 | 106.00 | 110.00 | 144.00 |
| | Percentiles | 25 | 29.0000 | 26.0000 | 18.0000 | 17.2500 | 20.0000 | 23.0000 |
| | | 50 | 36.0000 | 40.0000 | 32.0000 | 22.5000 | 22.0000 | 25.0000 |
| | | 75 | 108.0000 | 74.0000 | 46.0000 | 61.0000 | 38.0000 | 30.0000 |
| B | N | Valid | 6 | 6 | 7 | 7 | 7 | 7 |
| | | Lost | 1 | 1 | 0 | 0 | 0 | 0 |
| | Mean | | 27.9500 | 38.8333 | 43.4286 | 33.8857 | 38.2286 | 33.2143 |
| | Median | | 19.2500 | 28.5000 | 25.0000 | 23.0000 | 23.0000 | 24.5000 |
| | Std. dev. | | 25.27795 | 22.24785 | 49.35199 | 22.87702 | 31.14534 | 27.45277 |
| | Min. | | 11.00 | 20.00 | 13.00 | 15.00 | 13.00 | 15.00 |
| | Max. | | 78.00 | 74.00 | 151.00 | 72.20 | 99.60 | 94.00 |
| | Percentiles | 25 | 11.9000 | 22.2500 | 16.0000 | 19.0000 | 18.0000 | 19.0000 |
| | | 50 | 19.2500 | 28.5000 | 25.0000 | 23.0000 | 23.0000 | 24.5000 |
| | | 75 | 40.5000 | 62.7500 | 53.0000 | 61.0000 | 60.0000 | 34.0000 |

• The level of GPT observed in different times for group B (p=0.004):

| **Group** | | | **GPT T0** | **GPT T1** | **GPT T2** | **GPT T3** | **GPT T4** | **GPT T5** |
|---|---|---|---|---|---|---|---|---|
| A | N | Valid | 7 | 7 | 7 | 7 | 7 | 7 |
| | | Lost | 0 | 0 | 0 | 0 | 0 | 0 |
| | Mean | | 51.0000 | 45.0000 | 40.2857 | 32.7143 | 30.5714 | 30.2857 |
| | Median | | 36.0000 | 32.0000 | 27.0000 | 31.0000 | 31.0000 | 27.0000 |
| | Std. dev. | | 54.22791 | 46.54747 | 29.78654 | 21.06114 | 19.26012 | 21.47645 |
| | Min. | | 7.00 | 7.00 | 8.00 | 5.00 | 9.00 | 10.00 |
| | Max. | | 167.00 | 143.00 | 79.00 | 56.00 | 59.00 | 73.00 |
| | Percentiles | 25 | 15.0000 | 16.0000 | 15.0000 | 15.0000 | 12.0000 | 13.0000 |
| | | 50 | 36.0000 | 32.0000 | 27.0000 | 31.0000 | 31.0000 | 27.0000 |
| | | 75 | 60.0000 | 55.0000 | 76.0000 | 54.0000 | 49.0000 | 40.0000 |
| B | N | Valid | 6 | 6 | 7 | 7 | 7 | 7 |
| | | Lost | 1 | 1 | 0 | 0 | 0 | 0 |
| | Mean | | 15.7167 | 25.5000 | 22.2857 | 16.2143 | 16.2286 | 12.2857 |
| | Median | | 14.0000 | 19.5000 | 20.0000 | 15.0000 | 17.0000 | 12.0000 |
| | Std. dev. | | 9.56147 | 18.09696 | 12.44607 | 7.78812 | 7.95188 | 7.04070 |
| | Min. | | 5.50 | 12.00 | 11.00 | 7.00 | 8.00 | 4.00 |
| | Max. | | 33.50 | 60.00 | 43.00 | 28.00 | 31.00 | 23.00 |
| | Percentiles | 25 | 9.6250 | 12.7500 | 12.0000 | 10.0000 | 9.0000 | 4.0000 |
| | | 50 | 14.0000 | 19.5000 | 20.0000 | 15.0000 | 17.0000 | 12.0000 |
| | | 75 | 20.6000 | 36.7500 | 36.0000 | 22.0000 | 19.0000 | 18.0000 |

• The level of GGT observed in different times for group A (p=0.079):

| **Group** | | | **GGT T0** | **GGT T1** | **GGT T2** | **GGT T3** | **GGT T4** | **GGT T5** |
|---|---|---|---|---|---|---|---|---|
| A | N | Valid | 7 | 7 | 7 | 7 | 7 | 7 |
| | | Lost | 0 | 0 | 0 | 0 | 0 | 0 |
| | Mean | | 150.2857 | 131.7143 | 130.2857 | 142.4286 | 147.7143 | 240.8571 |
| | Median | | 68.0000 | 56.0000 | 69.0000 | 74.0000 | 98.0000 | 163.0000 |
| | Std. dev. | | 158.16206 | 144.03786 | 140.32904 | 160.26526 | 134.40575 | 204.12858 |
| | Min. | | 33.00 | 28.00 | 29.00 | 33.00 | 34.00 | 39.00 |
| | Max. | | 418.00 | 377.00 | 398.00 | 481.00 | 432.00 | 610.00 |
| | Percentiles | 25 | 37.0000 | 30.0000 | 34.0000 | 46.0000 | 76.0000 | 112.0000 |
| | | 50 | 68.0000 | 56.0000 | 69.0000 | 74.0000 | 98.0000 | 163.0000 |
| | | 75 | 335.0000 | 296.0000 | 246.0000 | 210.0000 | 190.0000 | 436.0000 |
| B | N | Valid | 6 | 6 | 7 | 7 | 7 | 7 |
| | | Lost | 1 | 1 | 0 | 0 | 0 | 0 |
| | Mean | | 94.9333 | 94.6667 | 81.7143 | 119.1429 | 190.0000 | 176.5714 |
| | Median | | 108.0000 | 79.0000 | 83.0000 | 87.0000 | 110.0000 | 120.0000 |
| | Std. dev. | | 50.15283 | 53.64202 | 32.55618 | 89.37455 | 179.62090 | 183.81227 |
| | Min. | | 31.60 | 32.00 | 39.00 | 40.00 | 37.00 | 37.00 |
| | Max. | | 144.00 | 191.00 | 140.00 | 281.00 | 476.00 | 554.00 |
| | Percentiles | 25 | 35.6500 | 64.2500 | 51.0000 | 41.0000 | 40.0000 | 54.0000 |
| | | 50 | 108.0000 | 79.0000 | 83.0000 | 87.0000 | 110.0000 | 120.0000 |
| | | 75 | 141.7500 | 131.7500 | 94.0000 | 193.0000 | 396.0000 | 271.0000 |

• If direct differences between groups A and B in each time point are compared independently (Mann-Whitney test for independent samples), there are differences between A and B in the levels of red blood cells (p=0.026), hemoglobin (p=0.038), and hematocrit (p=0.053) in time T2.
• In the levels of lymphocytes, there are differences in all the times:

| | **lymphocytes 10_3 ml T0** | **lymphocytes (%) T1** | **lymphocytes (%) T2** | **lymphocytes (%) T3** | **lymphocytes (%) T4** | **lymphocytes (%) T5** |
|---|---|---|---|---|---|---|
| Mann-Whitney U | 11.000 | 9.500 | 6.500 | 6.000 | 10.000 | 9.000 |
| Wilcoxon W | 39.000 | 37.500 | 34.500 | 34.000 | 38.000 | 37.000 |
| Z | -1.727 | -1.919 | -2.302 | -2.366 | -1.853 | -1.981 |
| Asym. sig. (2-sided) | **0.084** | **0.055** | **0.021** | **0.018** | **0.064** | **0.048** |
| Exact sig. [2*(1-sided sig)] | 0.097a | 0.053a | 0.017a | 0.017a | 0.073a | 0.053a |

• There are differences between A and B in the levels of neutrophils in T2 (p=0.007) and T3 (p=0.011)
• There are differences between A and B in the levels of GPT in T5 (p=0.038)
• There are no significant differences in the levels of urea over time, said levels remaining virtually the same in both groups (Friedman test, p=0.205 for group A and p=0.959 for group B):

### UREA

| **Group** | | | | **urea T0** | **urea T1** | **urea T2** | **urea T3** | **urea T4** | **urea T5** |
|---|---|---|---|---|---|---|---|---|---|
| A | | N | Valid | 7 | 7 | 7 | 7 | 7 | 7 |
| | | | Lost | 0 | 0 | 0 | 0 | 0 | 0 |
| | | | Mean | 51.4286 | 47.7143 | 45.0000 | 39.4286 | 40.2857 | 43.5714 |
| | | | Median | 57.0000 | 61.0000 | 60.0000 | 57.0000 | 54.0000 | 55.0000 |
| | | | Std. dev. | 26.13973 | 27.13985 | 28.56571 | 25.81897 | 30.05946 | 31.57455 |
| | | | Min. | 9.00 | 500 | 10.00 | 7.00 | 2.00 | 4.00 |
| | | | Max. | 83.00 | 74.00 | 77.00 | 67.00 | 72.00 | 79.00 |
| | Percentiles | | 25 | 23.0000 | 19.0000 | 14.0000 | 11.0000 | 8.0000 | 10.0000 |
| | | | 50 | 57.0000 | 61.0000 | 60.0000 | 57.0000 | 54.0000 | 55.0000 |
| | | | 75 | 69.0000 | 73.0000 | 71.0000 | 58.0000 | 66.0000 | 78.0000 |
| B | | N | Valid | 7 | 7 | 7 | 7 | 7 | 7 |
| | | | Lost | 0 | 0 | 0 | 0 | 0 | 0 |
| | | | Mean | 111.2857 | 115.0000 | 109.0000 | 114.2857 | 117.5714 | 115.7143 |
| | | | Median | 117.0000 | 120.0000 | 92.0000 | 100.0000 | 90.0000 | 99.0000 |
| | | | Std. dev. | 37.94168 | 33.78856 | 46.38965 | 54.75921 | 73.16160 | 85.85397 |
| | | | Min. | 62.00 | 68.00 | 66.00 | 69.00 | 4400 | 30.00 |
| | | | Max. | 169.00 | 172.00 | 191.00 | 213.00 | 250.00 | 260.00 |
| | Percentiles | | 25 | 64.0000 | 84.0000 | 71.0000 | 71.0000 | 61.0000 | 55.0000 |
| | | | 50 | 117.0000 | 120.0000 | 92.0000 | 100.0000 | 90.0000 | 99.0000 |
| | | | 75 | 133.0000 | 130.0000 | 150.0000 | 166.0000 | 182.0000 | 208.0000 |

• However, if the levels of urea between the two groups are compared in each measured time point, the levels of urea are higher in group B in all the times measured with the exception of the last. The differences are statistically significant:

| **Group** | | **N** | **Mean** | **Std. deviation** | **Std. error of the mean** | **p** |
|---|---|---|---|---|---|---|
| urea T0 | A | 7 | 51.4286 | 26.13973 | 9.87989 | |
| | B | 7 | 111.2857 | 37.94168 | 14.34061 | **0.011** |
| urea T1 | A | 7 | 47.7143 | 27.13985 | 10.25790 | |
| | B | 7 | 115.0000 | 33.78856 | 12.77087 | **0.002** |
| urea T2 | A | 7 | 45.0000 | 28.56571 | 10.79682 | |
| | B | 7 | 109.0000 | 46.38965 | 17.53364 | **0.004** |
| urea T3 | A | 7 | 39.4286 | 25.81897 | 9.75865 | |
| | B | 7 | 114.2857 | 54.75921 | 20.69704 | **0.001** |
| urea T4 | A | 7 | 40.2857 | 30.05946 | 11.36141 | |
| | B | 7 | 117.5714 | 73.16160 | 27.65248 | **0.026** |
| urea T5 | A | 7 | 43.5714 | 31.57455 | 11.93406 | |
| | B | 7 | 115.7143 | 85.85397 | 32.44975 | 0.073 |

On the other hand, the time evolution of the following parameters in each group separately has not shown significant differences:

### CREATININE

• There are no significant differences in the levels of creatinine over time, said levels remaining the same in both groups (Friedman test, p=0.122 for group A and p=0.831 for group B):

| **Group** | | | **creatinine T0** | **creatinine T1** | **creatinine T2** | **creatinine T3** | **creatinine T4** | **creatinine T5** |
|---|---|---|---|---|---|---|---|---|
| A | N | Valid | 7 | 7 | 7 | 7 | 7 | 7 |
| | | Lost | 0 | 0 | 0 | 0 | 0 | 0 |
| | | Mean | 1.7614 | 1.4957 | 1.3343 | 1.1929 | 1.1300 | 1.0571 |
| | | Median | 1.4400 | 1.1000 | 0.9700 | 1.1200 | 1.2200 | 0.7600 |
| | | Std. dev. | 1.15338 | 0.99052 | 0.90526 | 0.79229 | 0.61709 | 0.61302 |
| | | Min. | 0.49 | 0.48 | 0.46 | 0.39 | 0.46 | 0.40 |
| | | Max. | 3.70 | 2.80 | 2.47 | 2.18 | 2.05 | 1.94 |
| Percentiles | | 25 | 0.7500 | 0.6800 | 0.5000 | 0.4100 | 0.5200 | 0.5900 |
| | | 50 | 1.4400 | 1.1000 | 0.9700 | 1.1200 | 1.2200 | 0.7600 |
| | | 75 | 2.9100 | 2.7400 | 2.2200 | 1.9500 | 1.6700 | 1.7100 |
| B | N | Valid | 7 | 7 | 7 | 7 | 7 | 7 |
| | | Lost | 0 | 0 | 0 | 0 | 0 | 0 |
| | | Mean | 2.1157 | 2.1000 | 1.8843 | 1.8357 | 1.7600 | 1.5829 |
| | | Median | 2.0400 | 1.9700 | 1.9600 | 1.4100 | 1.6900 | 1.7200 |
| | | Std. dev. | 1.11884 | 0.84766 | 0.79521 | 0.93259 | 0.99698 | 1.05880 |
| | | Min. | 0.54 | 0.80 | 0.64 | 0.67 | 0.54 | 0.39 |
| | | Max. | 4.18 | 3.47 | 2.85 | 3.10 | 2.77 | 2.89 |
| Percentiles | | 25 | 1.6000 | 1.5800 | 1.1900 | 1.2300 | 0.7600 | 0.6100 |
| | | 50 | 2.0400 | 1.9700 | 1.9600 | 1.4100 | 1.6900 | 1.7200 |
| | | 75 | 2.6300 | 2.7000 | 2.7400 | 2.8900 | 2.7600 | 2.8200 |

• If the levels of creatinine between the two groups are compared in each measured time point, it is observed that there are no statistically significant differences in the levels of creatinine between groups A and B:

| **Group** | | **N** | **Mean** | **Std. deviation** | **Std. error of the mean** | **p** |
|---|---|---|---|---|---|---|
| creatinine T0 | A | 7 | 1.7614 | 1.15338 | 0.43594 | |
| | B | 7 | 2.1157 | 1.11884 | 0.42288 | 0.456 |
| creatinine T1 | A | 7 | 1.4957 | 0.99052 | 0.37438 | |
| | B | 7 | 2.1000 | 0.84766 | 0.32039 | 0.318 |
| creatinine T2 | A | 7 | 1.3343 | 0.90526 | 0.34216 | |
| | B | 7 | 1.8843 | 0.79521 | 0.30056 | 0.318 |
| creatinine T3 | A | 7 | 1.1929 | 0.79229 | 0.29946 | |
| | B | 7 | 1.8357 | 0.93259 | 0.35249 | 0.165 |
| creatinine T4 | A | 7 | 1.1300 | 0.61709 | 0.23324 | |
| | B | 7 | 1.7600 | 0.99698 | 0.37682 | 0.209 |
| creatinine T5 | A | 7 | 1.0571 | 0.61302 | 0.23170 | |
| | B | 7 | 1.5829 | 1.05880 | 0.40019 | 0.318 |

### LDH

• There are no significant differences in the levels of LDH overtime, said levels remaining virtually the same in both groups (Friedman test, p=0.355 for group A and p=0.921 for group B):

| | | **Group** | **LDH T0** | **LDH T1** | **LDH T2** | **LDH T3** | **LDH T4** | **LDH T5** |
|---|---|---|---|---|---|---|---|---|
| A | N | Valid | 7 | 7 | 7 | 7 | 7 | 7 |
| | | Lost | 0 | 0 | 0 | 0 | 0 | 0 |
| | | Mean | 708.5714 | 685.8571 | 638.0000 | 658.7143 | 602.4286 | 707.8571 |
| | | Median | 561.0000 | 631.0000 | 418.0000 | 499.0000 | 380.0000 | 552.0000 |
| | | Std. dev. | 345.48992 | 345.16538 | 474.81435 | 445.02723 | 458.08255 | 354.42462 |
| | | Min. | 504.00 | 320.00 | 327.00 | 377.00 | 364.00 | 397.00 |
| | | Max. | 1474.00 | 1369.00 | 1677.00 | 1659.00 | 1623.00 | 1421.00 |
| | Percentiles | 25 | 524.0000 | 421.0000 | 405.0000 | 480.0000 | 367.0000 | 491.0000 |
| | | 50 | 561.0000 | 631.0000 | 418.0000 | 499.0000 | 380.0000 | 552.0000 |
| | | 75 | 713.0000 | 836.0000 | 714.0000 | 570.0000 | 556.0000 | 912.0000 |
| B | N | Valid | 6 | 7 | 7 | 7 | 7 | 7 |
| | | Lost | 1 | 0 | 0 | 0 | 0 | 0 |
| | | Mean | 430.6667 | 591.4286 | 580.2857 | 570.5714 | 544.4286 | 609.5714 |
| | | Median | 420.0000 | 565.0000 | 545.0000 | 532.0000 | 590.0000 | 537.0000 |
| | | Std. dev. | 156.18024 | 142.31989 | 189.49557 | 159.89982 | 187.55964 | 257.69805 |
| | | Min. | 197.00 | 359.00 | 391.00 | 329.00 | 305.00 | 351.00 |
| | | Max. | 617.00 | 781.00 | 979.00 | 811.00 | 789.00 | 1066.00 |
| | Percentiles | 25 | 306.5000 | 540.0000 | 468.0000 | 465.0000 | 343.0000 | 408.0000 |
| | | 50 | 420.0000 | 565.0000 | 545.0000 | 532.0000 | 590.0000 | 537.0000 |
| | | 75 | 594.5000 | 756.0000 | 611.0000 | 697.0000 | 683.0000 | 819.0000 |

• If the levels of LDH between the two groups are compared in each measured time point, the levels of LDH are higher in group A in all the times measured, although the differences are not statistically significant:

| | **Group** | **N** | **Mean** | **Std. deviation** | **Std. error of the mean** | **p** |
|---|---|---|---|---|---|---|
| LDH T0 | A | 7 | 708.5714 | 345.48992 | 130.58292 | 0.073 |
| | B | 6 | 430.6667 | 156.18024 | 63.76032 | |
| LDH T1 | A | 7 | 685.8571 | 345.16538 | 130.46025 | 0.805 |
| | B | 7 | 591.4286 | 142.31989 | 53.79186 | |
| LDH T2 | A | 7 | 638.0000 | 474.81435 | 179.46296 | 0.535 |
| | B | 7 | 580.2857 | 189.49557 | 71.62259 | |
| LDH T3 | A | 7 | 658.7143 | 445.02723 | 168.20448 | 0.710 |
| | B | 7 | 570.5714 | 159.89982 | 60.43645 | |
| LDH T4 | A | 7 | 602.4286 | 458.08255 | 173.13893 | 0.710 |
| | B | 7 | 544.4286 | 187.55964 | 70.89088 | |
| LDH T5 | A | 7 | 707.8571 | 354.42462 | 133.95991 | 0.620 |
| | B | 7 | 609.5714 | 257.69805 | 97.40071 | |

### Example 4. Clinical trial protocol for COVID-19

### 4.1. Name and description of the investigational medication

Melatonin, injectable solution at high doses.

### 4.2. Summary of the clinical and non-clinical study findings that may be relevant for the trial.

Melatonin (N-acetyl-5-methoxytryptamine) is an indolamine synthesized from tryptophan through serotonin. It was first discovered as a product of the pineal gland in the brain which is produced rhythmically with a secretion peak or acrophase between 2 and 4 am. Melatonin synthesis is regulated by the biological clock and responsible for synchronizing circadian rhythms such as sleep/wakefulness, activity/rest, brain neurotransmitters, hormone rhythms, metabolic rhythms, etc. However, it is now known that melatonin is also produced in most, if not all, organs and tissues of the human body through the same synthesis pathway. Unlike pineal melatonin, the synthesis of this so-called extrapineal melatonin does not follow a circadian rhythm and is produced at much higher concentrations than pineal melatonin.

These high intracellular concentrations of melatonin confer a very effective antioxidant and anti-inflammatory potential thereto, while acting at the same time as mitochondrial protector enhancing ATP production for cellular defense. The antioxidant capacity of melatonin is two-fold: on one hand, it is a direct free radical scavenger that, however, also induces the expression and activity of antioxidant enzymes (superoxide dismutase, catalase, glutathione peroxidase and reductase, and glucose-6-phosphate dehydrogenase), as well as promotes the synthesis of glutathione, one of the main intracellular antioxidants, reducing markers of oxidative damage such as lipid peroxidation. Its anti-inflammatory activity depends on its two-fold capacity to inhibit the response of an exaggerated NF-kB- and NLRP3 inflammasome-dependent innate immune response, such as that which occurs in inflammatory processes like sepsis (see examples in the present specification). Melatonin also activates NK cells and reduces the total number of circulating PMNs.

In the event of viral infections, melatonin acts as a potent antiviral agent in experimental models. Like in sepsis, immune response can cause more damage than benefit in serious viral infections such as those caused by SARS-CoV, SARS-CoV-2, MERS, Ebola, and other related viruses. In 2012, a review by Boga *et*. *al.* (Boga JA, Coto-Montes A, Rosales-Corral SA, Tan DX, Reiter RJ. Beneficial actions of melatonin in the management of viral infections: a new use for this "molecular handyman". Rev Med Virol 2012;22:323-38.) summarizes the antiviral mechanisms of melatonin in different experimental infections, due to its effects in enhancing immune surveillance and eliminating free radicals, reducing cellular damage. In the case of the Ebola virus, infection promotes the destruction of vascular endothelium and the production of disseminated intravascular coagulation. The destruction of endothelium results from a cascade of inflammatory reactions, the activation of macrophages, monocytes, and the release of cytokines with excessive generation of oxygen and nitrogen free radicals. The aforementioned properties of melatonin are aimed at controlling those uncontrolled responses.

In the case of SARS-CoV-2, the pathogenic mechanisms involved include the binding of SARS-CoV-2 to ACE2 (angiotensin-converting enzyme II). This protein protects the lung by activating anti-atrophic, antifibrotic, antioxidant, and anti-inflammatory responses. The virus disables said functions enabling it to penetrate the cell and causing the respiratory symptoms of COVID-19. However, furthermore, upon disabling ACE2, AT1R (angiotensin 2 receptor type 1) which promotes pro-atrophic, profibrotic, pro-oxidative, and pro-inflammatory responses is activated, further favoring tissue damage. In this line of infection, once lung defense is stopped, cathepsins which are proteins with proteolytic activity are activated, favoring the formation of endosomes and the entry of the virus into the cell. **Based on this data, it can be considered that, the antioxidant and anti-inflammatory properties of melatonin on one hand, and its activity in inhibiting cathepsins on the other hand, suggest a promising role of melatonin against COVID-19 at specific doses.**

### 4.3 Description and justification of the route of administration, dose, dosage regimen, and treatment period

The properties of melatonin for its administration in this trial were obtained from different studies in experimental models, as well as from earlier clinical data. It is concluded based on those studies that the anti-inflammatory and antioxidant effects of melatonin are exerted at high doses of melatonin. These doses are due to the fact that melatonin, in order to exert its anti-inflammatory and antioxidant potential, must enter the cell which, as has been demonstrated, represents a barrier which prevents both the exit of intracellular melatonin to the outside and the entry of exogenous melatonin.

The doses were obtained from various experiments in animal models of sepsis and cancer, which allowed calculating the equivalent human dose ranging from 50 to 500 mg/day. With these past experiences and given the severity of COVID-19 patients showing a systemic inflammatory reaction resulting from the entry of the virus into the blood, similar to what occurs in sepsis, together with the respiratory failure that occurs later, it is considered that the doses of choice should be:
1) Treated group:
   Subgroup 1: Patients in ICU with respirator. A 60 mg/10 mL injectable melatonin solution is administered endovenously in a drip at a dose of 60 mg/6 h (10 mL of the solution/6 h), i.e., a total of 240 mg melatonin/day for a patient weighing <80 kg, and 120 mg/6 h (20 mL of the solution/6 h), i.e., a total of 480 mg melatonin/day for a patient weighing >80 kg. The treatment period will last from diagnosis and hospital admission until discharge. The level of efficacy will be assessed by comparing the number of discharges with melatonin compared to that achieved with the treatment indicated up until now.
   Subgroup 2: Patients hospitalized without respirator. A 60 mg/10 mL injectable melatonin solution is administered endovenously in a drip at a dose of 60 mg/8 h (10 mL of the solution/8 h), i.e., a total of 180 mg melatonin/day. The treatment period will last from diagnosis and hospital admission until discharge. The level of efficacy will be assessed by comparing the number of discharges with melatonin compared to that achieved with the treatment indicated up until now.
2) Placebo group:
   Subgroup 1: Patients in ICU with respirator. An injectable solution without melatonin (the same composition as that comprising melatonin) is administered endovenously in a drip at a dose of 10 mL/6 h, i.e., a total of 40 mL/placebo/day for a patient weighing <80 kg, and 20 mL/6 h, i.e., a total of 80 mL/placebo/ for a patient weighing >80 kg.
   Subgroup 2: Patients hospitalized without respirator. An injectable solution without melatonin (the same composition as that comprising melatonin) is administered endovenously in a drip at a dose of 10 mL/8 h, i.e., a total of 30 mL/placebo/day.

### 4.4. Description of the population to be studied

The patients to be studied comprise those diagnosed with SARS-CoV-2 infection and they will be classified into two groups:
1) A total of 180 patients (100 treated and 80 placebo) bedridden in UCI (serious or very serious, with respirator) between the ages of over 45 and 65 years, preferably over 55 years.
2) A total of 180 patients (100 treated and 80 placebo) admitted into hospital but not ICU (less serious or moderately serious, without respirator) between the ages of over 45 and 65 years, preferably over 55 years.

The inclusion criteria are:
1. Patients between the ages of over 45 and 65 years, preferably over 55 years, whose participation in the study is authorized by themselves or by family members and diagnosed of COVID-19.
2. Hospitalized, with or without supplemental oxygen or assisted ventilation.
3. With or without chronic or serious kidney failure.
4. With or without chronic or serious cardiovascular conditions.
5. With or without diabetes.
6. Immunosuppressed. Those derived from earlier classification and diagnosis, not biased by age or sex.

The exclusion criteria are:
1. Concomitant treatment which is incompatible with injectable melatonin.
2. Another experimental COVID19 treatment.
3. Liver transaminases > 5 folds the ULN.
4. Stage IV kidney failure or undergoing dialysis (GFR <30).
5. Allergy to melatonin.
6. Pregnancy.
7. Terminal medical or surgical disease.
8. Under treatment for psychiatric disease.
9. Under treatment with anticoagulants.
10. Refusal to participate in the study.

### 4.5. Bibliographic references and data which are relevant for the trial and provide justification for same

COVID-19 is the disease caused by the SARS_CoV-2 virus, an RNA-type β-coronavirus, originating presumably from bats, as indicated in the studies of the COVID-19 genome and bat CoV RaTG13. The human-human transmission occurs mainly between family members and friends which may come into contact with patients or healthy carriers.

The angiotensin-converting enzyme II (ACE2), found in the respiratory tract of humans, is SARS-CoV-2 receptor. The function of ACE2 is to transform angiotensin I to angiotensin 1-9 and angiotensin II to angiotensin 1-7. These products exhibit vasodilating, antifibrotic, anti-inflammatory effects and favor natriuresis. These effects reduce blood pressure, counter-regulating the action of angiotensin II. ACE2 has been linked to protection against HTA, arteriosclerosis, and other vascular and pulmonary processes. In contrast, the angiotensin-converting enzyme (ACE) which transforms angiotensin I to angiotensin II favors the generation of secondary peptides with vasoconstricting, pro-inflammatory, and sodium retaining effects, which are linked to the physiopathology of high blood pressure.

SARS-CoV-2 glycoprotein S is the main surface antigen with two S1 and S2 subunits, such that the distal protein S1 contains the ACE2 receptor binding domain, which prevents the natural protective activity thereof, whereas S2 contains the membrane fusion subunit to allow the entry of the virus into the target cell. This S protein is the one which causes immune responses during infection. The protein of the nucleocapsid, which is the most conserved antigen of the virus, activates the AP1 signal. This protein is recognized by immunoglobulins in the acute phase of infection and by surface T-cells of infected cells. The binding of the S protein to ACE2 is activated by TMPRSS2, a serine protein 2, as well as by cathepsin, which probably facilitates membrane invagination and the formation of endosome required for the entry of the virus into the host cell. Viral RNA, such as PAMP, is detected by PRR receptors, such as TLR3, TLR7, TLR8, and TLR9. From here on, retinoic acid receptor-inducible gene I (RIG-I) of the virus, melanoma differentiation associated gene 5 (MDA5), and the nucleotidyltransferase of cyclic GMP-AMP synthase (cGAS), are responsible for virus recognition. The intercellular signaling includes adaptor proteins such as TIR-domain-containing adaptor protein including INFβ (TRIF), mitochondrial antiviral-signaling protein (MAVS), and interferon gene-stimulating protein (STING). All of them trigger cascades of molecules including the adaptor protein MyD88, giving rise to the activation of NF-κB, which moves to the nucleus where it activates multiple pro-inflammatory and antioxidant signals, giving rise to an increase in IL-1, IL-2, IL-4, IL-7, IL-10, IL-12, IL-13, IL-17, GCSF, macrophage colony-stimulating factor (MCSF), IP-10, MCP-1, MIP-1α, hepatocyte growth factor (HGF), IFN-y, and TNF-α, causing an exaggerated immune response which is associated with the critical situation of COVID-19 patients.

The clinical characteristics imply that this virus is spread through the respiratory tract by respiratory droplets or secretions and direct contact. However, the fact that ACE2 is also found in the intestinal epithelium and that the virus has been isolated in the stool of some patients suggest that it is possible for the virus to enter through the digestive tract, in addition to the respiratory tract.

The most effective diagnostic method is through PCR from nasal or buccal swabs; rapid diagnostic methods are based on the presence of immunoglobulins IgM and IgG, which identify that there has been an infection, but not that the virus is active in the body.

Clinical symptoms include fever (88.7%), cough (67.8%), fatigue (38.1%), expectoration (33.4%), breathing difficulty (18.6%), and headache (13.6%). Furthermore, some patients exhibit gastrointestinal symptoms, with diarrhea (3.8%) and vomiting (5.0%). Susceptibility to infection increases with age, high blood pressure, chronic obstructive pulmonary disease, diabetes, and cardiovascular disease, whereas more severe complications include respiratory distress syndrome, septic shock, metabolic acidosis, coagulation disturbance, and multiorgan failure.

Laboratory analysis indicates that most patients have normal or low white blood cell counts with lymphocytopenia, although in severely ill patients, neutrophil counts, blood urea, and creatinine levels rise significantly, whereas lymphocytes continue to fall. Furthermore, inflammatory factors such as IL-6, IL-10, and TNF are elevated, reflecting the patients' immune status. ICU patients have higher levels of IL-2, IL-7, IL-10, GCSF, IP-10 (10 kD INFy-induced protein), MCP-1, MIP-1α, and TNFα.

Complications include acute respiratory stress syndrome, arrhythmia, shock, kidney failure, heart failure, liver dysfunction, and secondary infection. The disease progresses quicker in older patients, with the number of days from first symptom to death being shorter above the age of 65 years. Since there is no effective SARS-CoV-2 therapy, current treatments consist of symptomatic treatment and respiratory support. All patients accept oxygen therapy, and WHO recommends extracorporeal oxygenation in patients with refractory hypoxemia. Based on experience with other viral epidemics (SARS-CoV and MERS-CoV), systemic corticosteroids and some antivirals, neuraminidase inhibitors (oseltamivir, peramivir, zanamivir, etc.), ganciclovir, acyclovir, and ribavirin have been tested, but without results. Remdesivir has been effective in one case in the USA. In turn, chloroquine can inhibit pH-dependent viral replication, and suppress TNFα and IL-6, in addition to interfering with glycosylation of SASR-CoV cell receptors. Remdesivir (Gilead) is currently being used in a clinical trial in Spain on COVID-19 patients, and the use of remdesivir and chloroquine together against SARS-CoV-2 has been proposed.

On the other hand, for the present invention, both in experimental animals and in the human clinic, it has been verified how the therapeutic use of melatonin at suitable doses is capable of modulating immune response. The problem is that these viral diseases are self-limited by an adaptive immune response which depends on cell proliferation, and therefore requires several weeks to develop. In this window period, patients are vulnerable and mortality is high due to activation of innate immunity and concomitant oxidative stress. Controlling the innate immune response and reducing inflammation during this period increases patients' tolerance and decreases mortality in fatal virus infection.

Melatonin is a molecule which is capable of breaking this vicious cycle. Melatonin is a free radical scavenger powerful in reducing oxidative tissue damage and also an anti-inflammatory agent effective in curbing "cytokine storms" resulting from the activation of the NF-κB and NLRP3 inflammasome pathway. As a result, melatonin can increase host tolerance to pathogens and save precious time so that patients develop adaptive immune response and eventually recover from pathogen attack. Furthermore, melatonin also enhances adaptive immune response by increasing T-lymphocyte and B-cell proliferation to generate specific antibodies. Melatonin administration prevents septic shock and multiorgan failure by inhibiting iNOS expression and protecting mitochondria from the deleterious effect of excess nitric oxide.

The effect of melatonin was even greater in old than in young animals subjected to endotoxemia. In any case, melatonin prevented multiorgan failure in different tissues such as liver, lung, heart, diaphragm, etc. When the mechanisms of action of melatonin are analyzed to explain these outstanding anti-inflammatory effects, it is found that, on the one hand, melatonin acts by activating SIRT1 through the control of clock gene expression, binding to the nuclear receptor RORα; SIRT1, a deacetylase, deacetylates NF-κB in the nucleus, preventing it from binding to DNA and thereby ending its inflammatory activation. However, the other pathway of innate immunity, the NLRP3 inflammasome, continues to be active causing the pro-IL-1β produced by NF-κB binding to DNA to mature to its active form, maintaining the inflammatory response. NLRP3 inflammasome is activated by the presence of PAMPs, among others, and mitochondrial damage, induced by NF-κB pathway-dependent free radical (ROS) production. These ROS damage the mitochondria, open the transition pore, and release ROS and mitochondrial DNA into the cytosol, activating NLRP3 inflammasome. Melatonin acts as a potent mitochondrial protector, reduces ROS, closes the transition pore, blocking the inflammasome, which can no longer activate IL-1β. At the mitochondrial level, melatonin is much more potent than any other antioxidant, which favors its protection and ATP production, allowing the cell to have more ATP for its repair and protection functions.

All these actions, together with the effect of melatonin in counteracting respiratory dysfunction due to oxidative stress with age, as well as in protecting the myocardium from damage during aging and sepsis, make this molecule an exceptional therapeutic option for COVID-19.

### Example 5. EFFICACY AND SAFETY OF IV MELATONIN IN COVID-19 PATIENTS ADMITTED TO THE ICU

To conduct this study, all patients received standard treatment according to institutional protocols. Furthermore, patients were assigned randomly in a 2:1 ratio to receive:
- Experimental group (12 patients): 7 days of 5 mg/kg body weight/day of intravenous melatonin every 6 hours. Daily maximum dose of 500 mg a day.
- Control group (6 patients): 7 days of 5 mg/kg body weight/day of placebo with identical appearance through the intravenous route every 6 hours.

After 3 days of treatment, 3 intensive care physicians evaluated each participant and decided whether or not they wished to complete the treatment based on their clinical assessment:
- If there were objective or subjective signs of improvement or non-worsening of general clinical condition or respiratory failure, with no observation of inflammatory condition or multiorgan failure, the participant continued treatment until completion.
- If an adverse effect or clinical deterioration which can be objectively or subjectively attributed to the study drug was observed, treatment was discontinued.

The injectable composition was prepared according to the criteria established in Example 1 by adjusting the amounts to 5 mg/kg body weight/day of intravenous melatonin every 6 hours.

### Objectives

- To evaluate whether iv treatment with melatonin reduced the duration of ICU (intensive care unit) admission.
- To evaluate whether iv treatment with melatonin reduced the duration of mechanical ventilation (MV).
- To evaluate whether iv treatment with melatonin was associated with an increase in MV-free days.
- To evaluate whether iv treatment with melatonin is associated with a reduction in systemic inflammatory response, defined by the levels of ferritin, D-dimer, C-reactive protein (CRP) or procalcitonin (PCT) and IL-6.

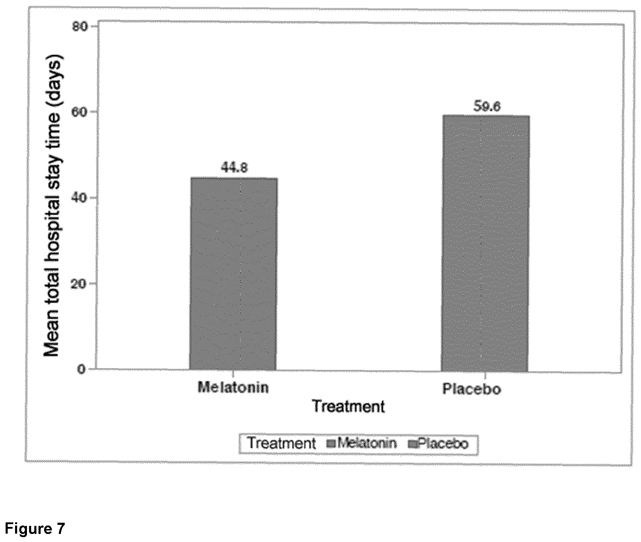

### 1. Evaluating whether iv treatment with melatonin reduced the duration of ICU (intensive care unit) admission (see Figure 7)

In the present clinical trial, no significant difference is observed in the duration of ICU admission for patients treated with melatonin compared to the duration of patients treated with placebo. However, it is observed that the duration of hospital admission is significantly shorter in patients treated with melatonin than in patients treated with placebo. There is a 15-day reduction in hospital stay for patients treated with melatonin compared to placebo (Figure 7)

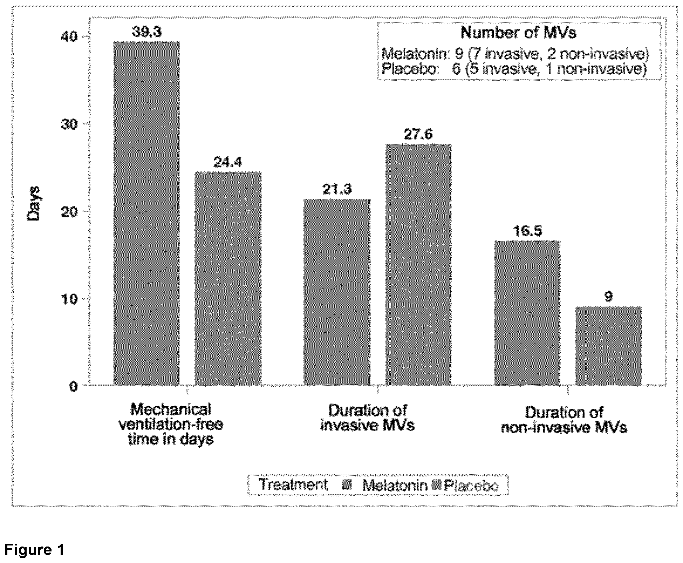

### 2. Evaluating whether iv treatment with melatonin reduced the duration of mechanical ventilation (MV) (Figure 1).

It was observed in the present trial that the duration of the invasive MV was significantly shorter for patients treated with melatonin compared to the duration of patients treated with placebo. Invasive mechanical ventilation was 21 days for the melatonin group and 30 days for placebo, whereas non-invasive mechanical ventilation was 16.5 days for the melatonin group and 9 days for the placebo group.

### 3. Evaluating whether iv treatment with melatonin is associated with an increase in MV-free days (see Figure 1)

In the present clinical trial it is observed that MV-free days is significantly higher for patients treated with melatonin compared to MV-free days for patients treated with placebo. During hospitalization, patients treated with melatonin were without mechanical ventilation for 40 days compared to 24 days without mechanical ventilation in the placebo.

### 4. Evaluating whether iv treatment with melatonin is associated with a reduction in systemic inflammatory response, defined by the levels of ferritin, D-dimer, C-reactive protein (CRP) or procalcitonin (PCT) and IL-6.

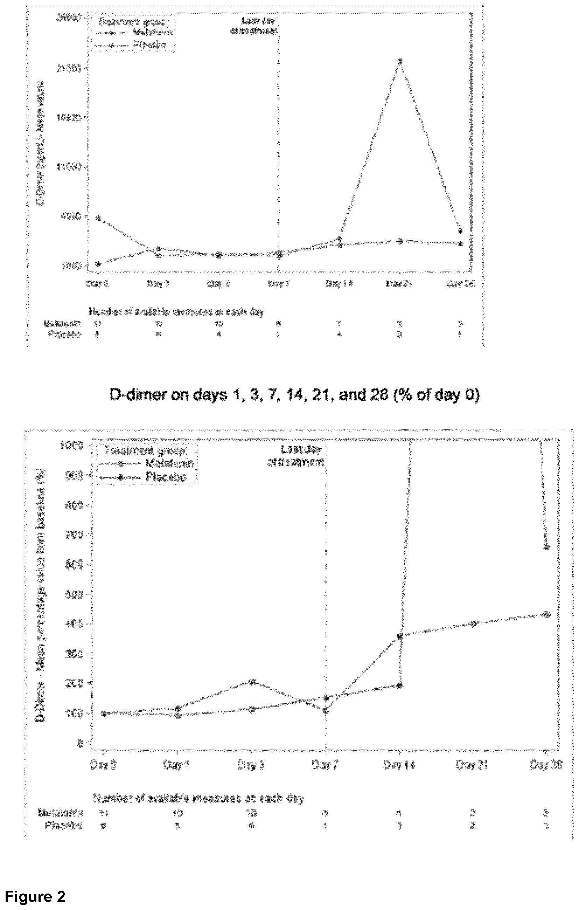

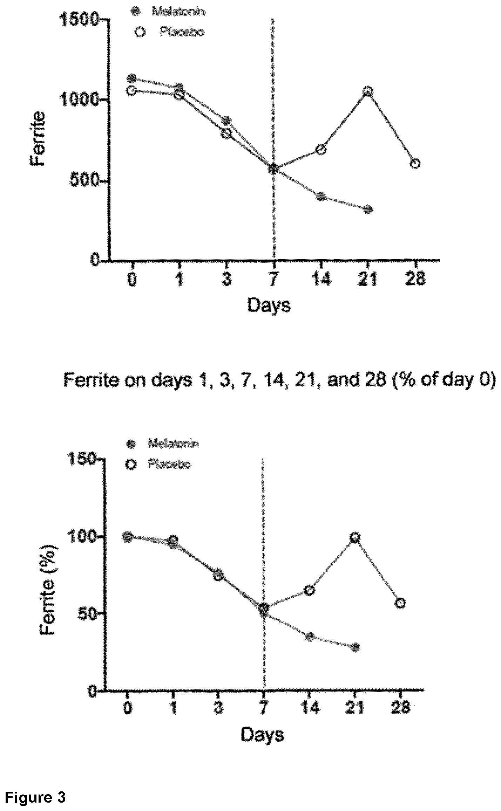

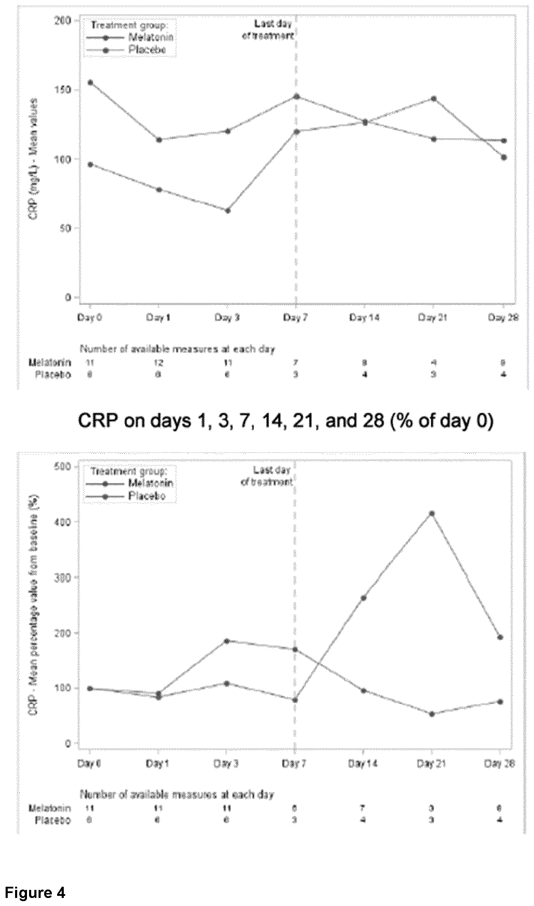

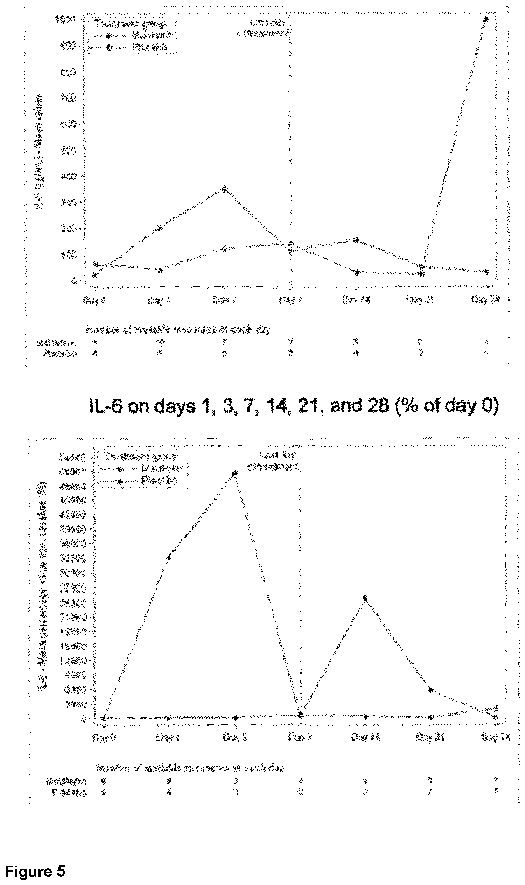

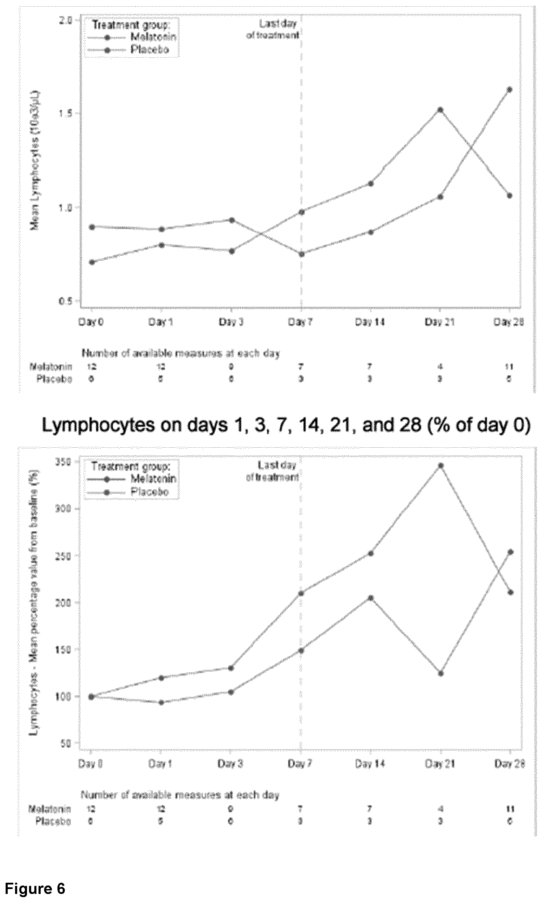

In the present trial, a reduction of the levels of dimer-D (Figure 2), ferritin or ferrite (Figure 3), CRP (Figure 4), as well as a reduction of IL6 (Figure 5), are observed during the days of treatment with melatonin. Likewise, an increase in lymphocytes (Figure 6) is observed during treatment with respect to placebo, whereas there is a tendency of neutrophils decreasing, and bilirubin has to drop. The data point to a reduction of humoral immunity and pro-inflammatory cytokines and an improvement of cellular immunity.

### Conclusions

The results of the present trial show that treatment with melatonin at a dose of 5 mg/kg/day for 7 days did not cause any adverse reactions, but rather caused an improvement in the inflammatory response. The significant reduction in total hospital stay for patients treated with melatonin (a 15-day reduction), as well as the significant reduction in invasive mechanical ventilation (a 9-day reduction) and the significant increase in the number of days without mechanical ventilation for said patients treated with melatonin (16 days) compared to placebo should be highlighted.

The decrease in inflammatory parameters such as ferritin, D-dimer, CRP, IL-6 and lymphocytes, point to a reduction of humoral immunity and proinflammatory cytokines and an improvement of cellular immunity.

## Claims

1. A pharmaceutically acceptable injectable composition comprising propylene glycol, polyethylene glycol, and melatonin or a derivative, a salt, a prodrug, or a solvate thereof, for use thereof in the treatment of viral infections such as SARS-CoV-2 at a minimum dose of 0.81 mg/kg/day by parenteral route.

2. The composition for use thereof according to claim 1, wherein said composition is used for the treatment of viral infections caused by SARS-CoV-2 in human subjects.

3. The composition for use thereof according to claim 2, wherein said composition is administered at a dose of melatonin or a derivative, a salt, a prodrug, or a solvate thereof, of between 0.81 mg/kg/day and 20 mg/kg/day by parenteral route.

4. The composition for use thereof according to claim 2, wherein said composition is administered at a dose of melatonin or a derivative, a salt, a prodrug, or a solvate thereof, of between 0.81 mg/kg/day and 9.80 mg/kg/day mg/day by parenteral route.

5. The composition for use thereof according to any of claims 3 or 4, wherein said composition comprises between 50 and 70 milligrams of melatonin or a derivative, a salt, a prodrug, or a solvate thereof, for every 10 ml of the total solution (w/v).

6. The composition for use thereof according to any of claims 3 or 4, wherein said composition comprises between 55 and 65 milligrams of melatonin or a derivative, a salt, a prodrug, or a solvate thereof, for every 10 ml of the total solution (w/v).

7. The composition for use thereof according to any of claims 3 or 4, wherein said composition comprises between 58 and 62 milligrams of melatonin or a derivative, a salt, a prodrug, or a solvate thereof, for every 10 ml of the total solution (w/v).

8. The composition for use thereof according to any of claims 3 or 4, wherein said composition comprises between 59 and 61 milligrams of melatonin or a derivative, a salt, a prodrug, or a solvate thereof, for every 10 ml of the total solution (w/v),

9. The composition for use thereof according to any of claims 3 or 4, wherein said composition comprises about 0.6 grams of melatonin or a derivative, a salt, a prodrug, or a solvate thereof, for every 100 ml of the total solution.

10. The composition for use thereof according to any of claims 5 to 9, wherein said composition is administered every 4, 6, or 8 hours.

11. The composition for use thereof according to any of the preceding claims, wherein said composition comprises water or a saline solution, propylene glycol, polyethylene glycol, and melatonin or a derivative, a salt, a prodrug, or a solvate thereof.

12. The composition for use thereof according to any of the preceding claims, wherein in said composition the proportion of propylene glycol is comprised between 5 and 50 grams for every 100 ml of the total solution (w/v).

13. The composition for use thereof according to any of claims 1 to 11, wherein in said composition the proportion of polyethylene glycol is comprised between 5 and 50 grams for every 100 ml of the total solution (w/v).

14. The composition for use thereof any of claims 1 to 11, wherein the proportion of propylene glycol is defined in claim 12 and the proportion of polyethylene glycol is defined in claim 13.

15. The composition for use thereof according to any of the preceding claims, wherein said composition is administered by intravenous route, preferably by perfusion.
